# EUROPEAN PATENT SPECIFICATION

(11) **EP 4 584 558 B1**
(45) Date of publication and mention of the grant of the patent: **29.04.2026**
(21) Application number: 23771910.9
(22) Date of filing: 05.09.2023
(51) Int. Cl.: G01F 1/46, G01F 7/00, A61B 5/087, A61B 5/091

(54) **PITOT TUBE FOR MEASURING HIGH AND LOW RESPIRATORY FLOW RATES**
PITOTROHR ZUR MESSUNG HOHER UND NIEDRIGER ATEMWEGSDURCHFLUSSRATEN
TUBE DE PITOT POUR LA MESURE DE DÉBITS RESPIRATOIRES ÉLEVÉS ET FAIBLES

(30) Priority: 05.09.2022 GB 202212901
(43) Date of publication of application: 16.07.2025
(73) Proprietor: Arete Medical Technologies Ltd, Cambridge, Cambridgeshire CB1 2LA (GB)
(72) Inventor: DOUGLAS, Graeham Rees, Cambridge Cambridgeshire CB1 2LA (GB); BOWKER-LONNECKER, Lin, Hamble Hampshire SO31 4HA (GB); WITHEY, Luke, Norwich Norfolk NR70AY (GB); MESSENGER, Richard, Launton Bicester Oxfordshire OX26 5AE (GB)
(74) Representative: Reddie & Grose LLP
(86) International application number: PCT/GB2023/052285
(87) International publication number: WO 2024/052656

(56) References cited:
- WO-A1-2021/218133
- CN-A- 103 070 686
- JP-A- H11 211 525
- US-A- 4 957 007
- US-A1- 2008 092 898
- US-A1- 2018 259 380

## Description

### Field of the invention

The invention relates to devices for performing one or more fluid flow tests and a method of using such a device. In particular, the invention relates to a device for performing a plurality of respiratory tests of different test types.

### Background to the invention

Devices for performing fluid flow tests are known. Such devices can comprise a housing defining a flow channel. Parameters of the flow of fluid through the flow channel can be measured using, for example, a flow sensor or a pressure sensor. Typically, devices for performing fluid flow tests are optimized for performing a particular type of fluid flow test having flow rates falling within an expected range. Such devices are often optimised for one type of flow test and are not suitable for performing other types of fluid flow test with flow rates falling outside the expected range. For example, a device that is optimized to measure flow during a fluid flow test with a relatively high flow rate may utilize an impeller within the flow, the impeller being connected to a counter. But an impeller-based design is typically unsuitable for measuring low flow rates accurately and repeatably. Another common technique for measuring flow is measuring a pressure drop across a fixed flow resistance, such as an orifice within a plate. However, for fluid flow tests with a relatively high flow and that require a low pressure drop in the flow channel, the use of an orifice may be unsuitable. An orifice hole may undesirably increase the pressure drop in the flow channel. Furthermore, an orifice hole can also cause noise or distortion in the flow which may be undesirable in some types of flow test.

An example of a device for performing a fluid flow test is a device for performing a respiratory test.

There are many people who suffer from conditions that affect their respiratory systems. These conditions can make it difficult to breathe and can have a negative impact day to day for those who suffer from them. In order to diagnose and monitor respiratory conditions in a subject, different tests can be performed which characterise various properties of the respiratory system. Often the results of more than one type of respiratory test are necessary to accurately diagnose or monitor a condition. Different respiratory tests have different expected ranges of fluid flow and different airflow requirements. Currently, devices that are optimized for performing a first respiratory test are generally not suitable for performing a second, different, respiratory test.

A first example of a respiratory test is a spirometry test. Peak flow rates during a spirometry test may be up to 16 litres per second. A second example of a respiratory test is a fractional exhaled nitric oxide (FeNO) test. FeNO testing requires a constant flow of 50 mL/s (± 10%), several orders of magnitude lower than the peak flow of a spirometry test.

Different considerations are required when designing a device for performing either of the above examples of respiratory tests. For example, measuring flow using an orifice in a plate, as described above, may be suitable for a device configured to perform an FeNO test. But the orifice creates a large pressure drop which is not suitable for a device performing a spirometry test. Spirometry tests require a low pressure drop in the flow channel through which a subject exhales.

On the other hand, an impeller may be suitable for measuring flow rate during a spirometry test but lacks the resolution and repeatability for measuring flow during FeNO tests..

Because devices configured to perform a particular type of respiratory test are not generally suitable for performing another type of respiratory test a number of separate devices are typically required to diagnose and monitor respiratory conditions, each device being optimized for performing a particular respiratory test. As the cost of individual devices can be high, current devices are often only available in specialist medical settings or lung function laboratories. Therefore, only a small portion of people suspected of having a respiratory disease are tested with such devices. It is similarly difficult to continually monitor symptoms over a series of testing sessions.

If a device for performing respiratory tests is to be reusable, any surfaces of the device which are exposed to airflow during use, including any sensing equipment, should be cleanable. This is because exhaled breath is nearly saturated with humidity, so moisture may condense in the device which needs removing after use. Furthermore, dust or other contaminants may also enter the device over time which also needs removing frequently. However, the cleaning agents or techniques that are most effective or efficient for cleaning the majority of the device's internal surfaces may cause damage to sensitive electronics or blockages of narrow sampling channels. Furthermore, cleaning agent could block a sampling channel, preventing a sensor from operating correctly until that droplet evaporated or dislodged from the channel.

It would be desirable to provide a device that is capable of accurately performing fluid flow tests over a larger range of expected flow rates than has been achieved previously, for example a range fluid flow rates that would be large enough for performing different types of flow tests. It would be desirable to provide a device for accurately performing a plurality of different fluid flow tests. It would be particularly desirable to provide a device for accurately performing respiratory tests. It would also be desirable to provide device for performing respiratory tests that is cleanable without damaging sensing equipment of the device. It would be desirable to provide such a device that is low cost and compact.

US 2018/259380 A1 discloses a flow meter comprising a sampling tube through which fluid may flow and a sensor arrangement. The sampling tube comprises a first hollow section having a first internal cross-sectional area and a second hollow section having a second internal cross-sectional area being less than the first internal cross-sectional area. The sensor arrangement, is for measuring the difference between stagnation and static pressures within the second hollow section. In particular, the sensor arrangement comprises a first pressure transducer for measuring a pressure difference between a first stagnation probe and a first static port, and a second pressure transducer for measuring a pressure difference between a second stagnation probe and a second static port.

### Summary of Invention

The invention provides a device for performing one or more fluid flow tests and a method for performing one or more fluid flow tests using the device according to the appended independent claims, to which reference should now be made. Preferred or advantageous features of the invention are defined in the dependent claims.

In a first aspect of the disclosure there is provided a device for performing one or more fluid flow tests. Preferably, the device is a device for performing a plurality of fluid flow tests of different types. The one or more fluid flow tests may be respiratory tests. Preferably, the device is for performing at least a first type of respiratory test and a second type of respiratory test. Preferably, the first type of respiratory test is different to the second type of respiratory test.

The device comprises a housing defining a flow channel having a distal end and a proximal end. Fluid flowing from the proximal end to the distal end of the flow channel flows in a first direction.

The device comprises a first pitot tube. The first pitot tube comprises a first port in fluidic communication with the flow channel. The first port faces in a second direction that is opposite to the first direction.

The device further comprises a second port in fluidic communication with the flow channel. The second port does not face in the second direction.

The device further comprises a first sensor assembly for measuring the difference between the pressure in the first pitot tube and the pressure at the second port and a second sensor assembly for measuring the difference between the pressure in the first pitot tube and the pressure at the second port. The first sensor assembly is sensitive to a range of pressure or differential pressure that is higher than the range that the second sensor assembly is sensitive to.

Because the first port of the first pitot tube faces in the second direction, the first port and the pitot tube may be exposed to the stagnation pressure created by fluid flowing the first direction.

As used herein, the "*stagnation pressure*" is the pressure measured at a stagnation point in the flow of fluid in the flow channel. The stagnation point is a point in the flow where the local velocity of the fluid is zero due to interaction of the flow with an obstruction. The stagnation pressure is the sum of the static and dynamic pressure. The stagnation pressure is the same as the total pressure.

The first pitot tube may be configured such that, when fluid flows through the flow channel in the first direction, a stagnation point may be created within the first pitot tube. This is because the fluid flowing through the flow channel in the first direction may enter the pitot tube through the first port which is in fluidic communication with the flow channel. The flow may then be brought to rest in the pitot tube. As such, the stagnation pressure may advantageously be measured by measuring the pressure in the first pitot tube.

Preferably, the first pitot tube comprises a housing. The housing of the first pitot tube may define an internal passage. The first port may be in fluidic communication with the internal passage. The first port may be an aperture defined in the housing of the first pitot tube. The aperture may extend through the housing of the first pitot tube to meet the internal passage.

Preferably, the internal passage extends in a straight line. The provision of a straight internal passage advantageously allows the pitot tube to be simpler and cheaper to manufacture at least because it is easy to injection mould such a pitot tube. Furthermore, a straight internal passage is easier to clean and reduces the risk of bacteria build-up which might be a problem if a more complicated geometry of passage where used. Preferably, the internal passage extends substantially perpendicularly to the first direction.

When fluid flows in the first direction, the stagnation point may be within the internal passage of the pitot tube. At least a first portion of the hollow tube may extend in a direction that is not parallel to the first direction. Preferably, the first portion of the hollow tube is perpendicular to the first direction. Preferably, the first portion of the hollow tube is downstream of the first port when fluid flows in the first direction.

The second port of the device does not face the flow of fluid when fluid flows in the first direction. As such, the second port may be exposed to the static pressure of fluid flowing the first direction, but not the dynamic pressure. The static pressure may therefore be measured at the second port.

As used herein, the "*static pressure*" is the portion of pressure in the flow channel in absence of the dynamic pressure.

For example, it is the pressure exerted on walls of the flow channel that are perpendicular to the flow direction. Due to frictional losses, the static pressure can be different axially along the flow channel. Therefore, it is advantageous for the first port and the second port to be located near to one another in the flow channel axis.

The first and second sensor assemblies are each configured for measuring the difference between the pressure in the first pitot tube and the pressure at the second port. When fluid flows in the first direction, the pressure in the first pitot tube may be the stagnation pressure and the pressure at the second port may be the static pressure. So, the difference between the pressure in the first pitot tube and the pressure at the second port may be the dynamic pressure. In other words, when fluid flow in the first direction, the first and second sensor assembly may each be configured for measuring the dynamic pressure of the fluid.

It may be particularly advantageous to remove the static pressure component of the stagnation pressure when the static pressure makes up a significant portion of the stagnation pressure and so cannot be ignored. This may be the case, for example, in respiratory tests.

The device may be for performing one or more fluid flow tests in which the output is a result based on the difference in pressure between the first pitot tube and the second port. In other words, the output may be based on a dynamic pressure value measured by the device.

As used herein, the result being "*based on the dynamic pressure value*" means that the result that is output may be equal to the dynamic pressure value or the result that is output may be calculated using the dynamic pressure value.

If the result is calculated using the dynamic pressure value, the calculation may comprise calibrating the dynamic pressure value. For example, the calibration may comprise multiplying the dynamic pressure value by a linear scale factor or fitting a higher-order calibration model to an external flow sensor across a series of input flows. The calibrated dynamic pressure value may be the velocity of the flow, the momentum of the flow, flow rate of the flow, or the kinetic energy of the flow. The method may comprise outputting the calibrated dynamic pressure value as the result of the first fluid flow test.

The device may comprise one or more additional sensors. The calculation of the result may comprise combining the dynamic pressure value with one or more other values measured using the one or more additional sensors of the device. For example, the first test may be an FeNO test. The one or more additional sensors may comprise at least one of an electrochemical sensor, a temperature sensor and a humidity sensor. The result of the FeNO test may be a concentration of nitric oxide in the air which may be calculated based on a value representing nitric oxide levels measured by an electrochemical sensor of the device as well as the dynamic pressure value. Furthermore, the result of the FeNO test may be calibrated using temperature and/or humidity values measured using temperature and humidity sensors of the device.

As used herein, the first port "*facing*" the second direction means that the first port is exposed to the stagnation pressure when fluid flows in the first direction and, in particular, the dynamic pressure component of the stagnation pressure. The first port facing in the second direction therefore means that the first port is facing fluid flowing towards the first port when fluid flows in the first direction. The first port may lie in the first plane. A normal of the first plane may not be perpendicular to the first direction. The angle between the first direction and the normal of the first plane may be greater than 90 degrees or less than 270 degrees. The angle may be defined in a clockwise direction and may be the angle needed to rotate from an axis defined by the first direction to the normal of the first plane. The normal of the first plane may be defined from the port into the flow chamber. The normal of the first plane may preferably be parallel to the first direction.

As used herein, the second port "*not facing*" the second direction means that the second port may only be exposed to the static pressure component of the stagnation pressure of the fluid flowing through the flow channel in the first direction. The second port not facing in the second direction therefore means that the second port is not facing fluid flowing towards the second port when fluid flows in the first direction. The second port may lie in the second plane. A normal of the second plane may be non-parallel to the first direction. The angle between the first direction and the normal of the second plane may be less than 90 degrees or greater than 270 degrees. The angle may be defined in a clockwise direction and may be the angle needed to rotate from the first direction to arrive at the normal of the second plane. The normal of the second plane may be defined from the port into the flow chamber. The angle between the first direction and the normal of the second plane may be less than 45 degrees or greater than 315 degrees. The normal of the second plane may be parallel to the first direction.

The use of a pitot tube for determining pressure may be advantageous for most flow tests. The use of a pitot tube for determining pressure has been found to be particularly advantageous for performing respiratory tests.

One reason that the use of a pitot tube may be particularly advantageous is that the pitot tube and flow channel may be configured such that the pitot tube causes a relatively small pressure drop in the flow channel. The pressure drop caused by the pitot tube may be negligible when compared to the pressure drop caused by an orifice in a plate, for example. So, a device comprising a pitot tube to measure pressure may be suitable for performing a high flow, low pressure drop test, such as a spirometry test.

Another reason that the use of a pitot tube is particularly advantageous is that the pressure in the pitot tube may change quickly when pressure in the flow channel changes. So, unlike impeller arrangements for example, the inherent design of the pitot tube may allow for accurate and responsive measurements to be made even at low or very low flow rates. The accuracy at low flow rates and responsiveness of the pressure measurements made using a pitot tube may advantageously be dependent on the sensitivity and responsiveness of the first and second sensor assembly. So, a device comprising a pitot tube to measure pressure may be suitable for performing a low flow test such as an FeNO test.

By providing a first sensor assembly that is sensitive to a higher range of pressure or differential pressure than the second sensor assembly, the device may advantageously be configured to accurately measure the pressure difference between the first pitot tube and the second port over a much wider range of pressures than if a single sensor assembly where provided. This is particularly advantageous when the device is for performing a plurality of flow test types. For example, a first flow test type may have an expected pressure range falling within the sensitive range of the first sensor assembly. A second flow test type may have an expected pressure range falling within the sensitive range of the second sensor assembly. The device may advantageously be able to perform both the first and second fluid tests, of differing test type, using the same flow channel and same pitot tube. In this way, a single device may be provided rather than a plurality of devices. This may advantageously reduce the amount of hardware and cost of performing those tests. This may be particularly advantageous in the context of respiratory tests. A device being configurable to perform more than one respiratory diagnostic test advantageously means that the single device allows for a fuller diagnosis or fuller monitoring of a respiratory condition.

Each of the first and second sensor assembly may comprise one or more pressure sensors. The range of pressures that each of the first and second sensor assemblies is sensitive to may depend on the range of the pressure sensor of the respective sensor assembly.

As used herein, the "*range of pressures*" that a sensor assembly or pressure sensor is sensitive to is a range defined between a maximum and minimum value that the sensor assembly or pressure sensor can accurately measure. Outside of this range, the sensor assembly or pressure sensor may output a constant value, significantly change sensitivity or have other erratic behaviour providing erratic results. The range may by stated by the manufacturer of the sensor.

The first sensor assembly may be sensitive to a maximum pressure or differential pressure that is at least two times greater, preferably at least three times greater, preferably at least four times greater, preferably at least five times greater, preferably at least an order of magnitude greater than the maximum pressure or differential that the second sensor assembly is sensitive to.

The first sensor assembly may be sensitive to a range of pressures that overlaps with the range of pressures that the second sensor assembly is sensitive to. Alternatively, the first sensor assembly may be sensitive to a range of pressures that does not overlap with the range of pressures that the second sensor assembly is sensitive to.

The first sensor assembly may comprise a differential pressure sensor. The differential pressure sensor of the first sensor assembly may be in fluidic communication with the first port and the second port. The differential pressure sensor may be configured to measure the differential pressure between the first pitot tube and the second port.

The first sensor assembly may be sensitive to a pressure or differential pressure of between 1000 Pa and 5000 Pa, preferably between 1000 Pa and 4000 Pa, more preferably between 1000 Pa and 3000 Pa, more preferably between 1250 Pa and 2750 Pa, most preferably between 1500 Pa and 2000 Pa.

The second sensor assembly may be sensitive to a pressure or differential pressure of between 10 Pa and 1000 Pa, preferably between 10 Pa and 800 Pa, preferably between 10 Pa and 500 Pa, preferably between 10 Pa and 500 Pa, preferably between 10 Pa and 500 Pa, preferably between 10 Pa and 250 Pa, more preferably between 10 Pa and 125 Pa.

The first and second sensor assemblies may have a similar or, preferably, identical operating principle to one another. For example, one or more of the following properties of the sensor assembly may be similar or identical: packaging style and size, the thermal properties, and the cross-sensitivities to factors other than pressure.

Advantageously, the two sensor assemblies may be identical to one another in every way, except in that one has a higher maximum sensitivity than the other and the other has a finer resolution. The second sensor assembly may comprise a differential pressure sensor. The differential pressure sensor of the second sensor assembly may be in fluidic communication with the first port and the second port. The differential pressure sensor may be configured to measure the differential pressure between the first pitot tube and the second port.

The differential pressure sensor of the first sensor assembly may sensitive to a higher range of pressure differentials than the differential pressure sensor of the second sensor assembly.

Alternatively, the first sensor assembly may comprise a first sensor configured to measure the pressure in the first pitot tube and a second sensor configured to measure the pressure at the second port. The control circuitry may be configured to determine the differential pressure based on the difference between the two measured pressures.

The second sensor assembly may comprise a first sensor configured to measure the pressure in the first pitot tube and a second sensor configured to measure the pressure at the second port. The control circuitry may be configured to determine the differential pressure based on the difference between the two measured pressures.

The first and second pressure sensors of the first sensor assembly may be sensitive to a higher range of pressures than the first and second pressure sensors of the second sensor assembly.

When the first and/or second sensor assemblies each comprise first and second pressure sensors, the device may comprise control circuitry configured to determine the difference between the pressure in the first pitot tube and the pressure at the second port based on the difference between a pressure measured by the respective first and second pressure sensor.

Preferably, the first sensor assembly comprises one or more sensors and the second sensor assembly comprises one or more sensors. The one or more sensors of the first sensor assembly may be different to the one or more sensors of the second sensor assembly.

The first sensor assembly may share a pressure sensor with the second sensor assembly. In such cases, the difference in the sensitivity of the sensor assemblies may arise from other electrical components of the sensor assembly. For example, the device may comprise an analog pressure sensor. The first sensor assembly may comprise the analog pressure sensor and a first analog to digital converter (first ADC). The second sensor assembly may comprise the analog pressure sensor and a second analog to digital converter (second ADC), the first ADC being different to the second ADC. Preferably, the first ADC is sensitive to a higher range of pressure signals from the first pressure sensor than the second ADC. In this way, the first sensor assembly is sensitive to a higher range of pressure or differential than the second sensor assembly.

The device may comprise a static port. The static port of the device may be the second port, as described above. When the device comprises a static port as the second port, the first and second sensor assembly may be configured to measure the difference between the pressure in the first pitot tube and the pressure at the static (second) port when air flows in the air flow channel in the first direction.

The static port may be defined in a portion of the housing that defines the flow channel. The static port may lie parallel to the first direction.

The static port may be closer to the mouthpiece than the first port. The static port may be less than 10, 9, 8, 7, 6, 5, 4, 3 or 2 centimetres from the mouthpiece.

Alternatively or additionally, the device may comprise a second pitot tube. The second pitot tube may comprise a port.

The port of the second pitot tube may be the second port, as described above. When the second pitot tube comprises the second port, the first and second sensor assembly may be configured to measure the difference between the pressure in the first pitot tube and the pressure in the second pitot tube.

Alternatively, if the device comprises a static port and a second pitot tube comprising a port, the port of the second pitot tube may be referred to as a third port. In such cases, the first and second sensor assembly may be configured to measure the difference between the pressure in the second pitot tube and the pressure at the static (second) port when air flows in the air flow channel in the second direction.

In the embodiment wherein the device comprises a static port, the first sensor assembly may comprise a first sensor configured to measure the pressure in the first pitot tube and a static sensor configured to measure the pressure at the static (second) port.

In the embodiment wherein the device comprises a static port, the second sensor assembly may comprise a second sensor configured to measure the pressure in the first pitot tube. The first sensor may be sensitive to a higher range of pressures than the second sensor, such that the first sensor assembly is sensitive to a higher range of pressures than the second sensor assembly.

In the embodiment wherein the device comprises a static (second) port and a second pitot tube comprising a third port, the device may comprise a third sensor assembly. The third sensor assembly may comprise a third sensor configured to measure the pressure in the second pitot tube in addition to the static sensor configured to measure the pressure at the static (second) port.

In the embodiment wherein the device comprises a static (second) port and a second pitot tube comprising a third port, the device may comprise a fourth sensor assembly. The fourth sensor assembly may comprise a fourth sensor configured to measure the pressure in the second pitot tube and the static sensor configured to measure the pressure at the static (second) port.

The third sensor may be sensitive to a higher range of pressures than the fourth sensor, such that the third sensor assembly is sensitive to a higher range of pressures than the fourth sensor assembly.

Preferably, the (second or third) port of the second pitot tube faces away from the second direction. This advantageously allows the dynamic pressure to be measured when fluid flows through the flow channel in the second direction opposite to the first direction, in addition to the first direction. In other words, the dynamic pressure of fluid flowing in the flow channel from the distal end to the proximal end may be measured when the device comprises a second pitot tube comprising the second port.

The second pitot tube comprising a (second or third) port may advantageously allow the device to perform fluid flow tests when fluid flows through the flow channel in either the first or second direction. For example, an spirometry test may be performed, at least in part, when a subject inhales through the flow channel. A device comprising the second pitot tube as defined above could advantageously be configured to perform different types of tests using the same set of pitot tubes and sensors.

When fluid flows in the second direction, the second port may face towards the direction of flow of fluid through the flow channel. The first port may face away from the direction of flow of fluid through the flow channel. The port of the second pitot tube may be exposed to the stagnation pressure. The first port may be exposed to static pressure. So, the first and second sensor assembly may measure the dynamic pressure by measuring the difference between the pressure in the first pitot tube and pressure in the second pitot tube.

The second pitot tube may comprise corresponding features to the first pitot tube. For example, the second pitot tube may comprise a second pitot tube housing. The housing of the second pitot tube may define an internal passage. The second port may be in fluidic communication with the internal passage. The second port may be an aperture defined in the housing of the second pitot tube. The aperture may extend through the housing of the second pitot tube to meet the internal passage.

The first pitot tube and the second pitot tube may be symmetrical to one another. This may be particularly advantageous when fluid may flow in both the first and second directions through the flow channel as the pitot tubes may have the same physical characteristics as one another.

A longitudinal axis may be defined through the centre of the flow channel. Preferably, the first port may be aligned with longitudinal axis. Preferably, the second port may be aligned with the longitudinal axis. Air flow may be least turbulent or have fewer localised flow pattern disturbances near the centre of the flow channel. So, aligning the first and/or second port with the longitudinal axis may provide pressure measurements made by the first and second sensor assemblies that are more consistent with the overall flow in the airflow channel.

The flow channel may be axisymmetric. The longitudinal axis may be the axis of symmetry of the axisymmetric flow channel.

A separation between the first port and the second pitot port along the longitudinal axis may be less than 5 centimetres, preferably less than 4 centimetres, preferably less than 3 centimetres, preferably less than 2 centimetres, preferably less than 1 centimetre. The first pitot tube may be in contact with the second pitot tube. As above, the static pressure can may vary axially along the flow channel. This may be due to frictional losses. Reducing the separation between the first and second port may reduce the difference between the true static pressure and the static pressure measured at the first or second port.

As used herein, the separation between the first and second pitot tubes means the shortest distance between the two pitot tubes. For example, when the first and second pitot tubes each comprise a housing, the separation may be the shortest distance between an external surface of the housing of the first pitot tube and an external surface of the housing of second pitot tube.

The distance between the first port and the distal end may be less than the distance between the second port and the distal end.

The distance between the first pitot tube and the proximal end of the flow channel may be substantially the same as the distance between the second pitot tube and the distal end of the flow channel.

The device may comprise a flow lineariser. The flow lineariser may comprise a material or component that is porous, woven, vaned, or having another structure that serves to disrupt locally swirling flows and aligns the local flow patterns with the bulk flow direction. The flow lineariser may be positioned within the flow channel. The flow lineariser may be configured to reduce vortices or turbulent fluid flow immediately upstream and/or immediately downstream of the first pitot tube when fluid flows in the first direction. The flow lineariser may be configured to reduce vortices or turbulent flow resulting from end effects at the distal end of the flow channel or the proximal end of the flow channel or other changes to the geometry of the walls of the flow channel. The device may comprise one or more flow linearisers.

As used herein, the terms '*upstream'* and '*downstream'* are used to describe the relative positions of components, or portions of components, of the device in relation to the direction of fluid flowing through the flow channel from the proximal end to the distal end.

It is preferable that, when fluid flows through the flow channel, the fluid flow at the first port and the second port is laminar. This is because turbulent flow at the first and/or second port may result in noise being generated in the pressure measurements made using the first and second sensor assemblies. It is desirable to reduce noise. The flow lineariser may advantageously reduce the turbulent fluid flow and so reduce noise.

One source of turbulent flow in the flow channel may be the first pitot tube (or second pitot tube, if present). When fluid flows in the first direction, it may pass the first pitot tube before reaching the second port. The first pitot tube may cause turbulence in the fluid flow which reaches the second port. The flow lineariser may advantageously reduce the turbulence generated by the first pitot tube. Preferably, a flow lineariser for reducing the turbulence generated may reduce turbulent flow immediately downstream of the first pitot tube.

The flow lineariser may comprise one or more elements extending from a position adjacent to the first pitot tube toward the distal end of the flow channel in a direction substantially parallel to the first direction. This may advantageously reduce turbulence downstream of the first pitot tube when fluid flows in the first direction.

Alternatively or additionally, the flow lineariser may comprise one or more elements extending from the first pitot tube toward the proximal end of the flow channel in a direction substantially parallel to the first direction. This may advantageously reduce turbulence upstream of the first pitot tube when fluid flows in the first direction.

The one or more elements may comprise or consist of one or more fins extending parallel to the first direction. Alternatively, the elements may comprise or consist of one or more tubes aligned parallel to the first direction. Alternatively or additionally, the elements may comprise or consist of a honeycomb structure.

The flow lineariser may comprise a walled structure defining a conduit. The wall of the walled structure may be relatively thin. A central axis of the conduit may extend in a direction substantially parallel to the first direction. A portion of the first pitot tube may be received within the conduit. The conduit may comprise two ends and may be open at both ends. The conduit may be configured such that fluid flowing through the flow channel also flows through the conduit. The conduit may be configured such that it is within the flow channel and most of the flow in the flow channel passes outside to the conduit.

Preferably, the first port may be received within the conduit. The conduit may advantageously reduce fluid turbulence created by the first pitot tube. Outside of the conduit, fluid flowing through the flow channel may be shielded from at least the portion of the first pitot tube that is received in the conduit. Inside of the conduit, turbulence generated by the portion of the first pitot tube received in the conduit may be reduced downstream of the first pitot tube (in the first direction).

Preferably, the conduit may have a width that is less than 50%, preferably less than 40%, preferably less than 30% the width of the flow channel. Generally, the smaller the width of a passage, the greater the extent to which turbulence in that passage is reduced. So, a conduit with a smaller width will reduce turbulence to a greater extent. A conduit with a smaller width relative to the width of the flow channel may have less flow resistance in the flow channel and the static pressure difference between the two ends of the conduit may be lower. For some respiratory tests such as spirometry, the flow channel flow resistance is preferably low. If the first port is nearer to one end of the conduit and the second port is nearer to the other end of the conduit, the static pressure difference caused by the flow resistance of the conduit in the flow channel is preferably low.

The conduit may extend downstream of the first pitot tube. The conduit may extend upstream of the first pitot tube.

The conduit may extend downstream of the first pitot tube such that the second port is received within the conduit. When device comprises a second pitot tube, a portion of the second pitot tube may be received within the conduit.

The conduit may comprise a first aperture through which the first pitot tube is receivable. The conduit may comprise a second aperture through which the second pitot tube is receivable.

The flow lineariser may comprise at least a portion of an external surface of the first pitot tube.

The external surface of the first pitot tube may comprise one or more axial projections, extending parallel to the first direction. One or more of the axial projections of the first pitot tube may extend downstream of the first pitot tube. One or more of the axial projections of the first pitot tube may extend upstream of the first pitot tube.

The portion of the external surface of the first pitot tube may be shaped such that a cross-section of the first pitot tube is elongate, the cross-section being defined by a plane having a normal that is perpendicular to the first direction. A length of the cross-section of the first pitot tube in a direction parallel to the first direction may be longer than a length of the cross-section of the first pitot tube in a direction perpendicular to the first direction, for example. Compared to other shapes, the cross-section of the first pitot tube having an elongate shape may advantageously smooth the flow of fluid in the flow channel and may advantageously reduce vortices.

The flow lineariser may be integrally formed with the flow channel and/or device housing.

The flow lineariser may comprise a mesh material surrounding the first port. By forcing the flow to pass through the voids and channels in a mesh material, vortices and other large swirling patterns in the flow can be aligned to smaller flow patterns, which more closely resemble a smooth or laminar flow pattern. The mesh structure may have a plurality of channels that align to be generally straight. Each of the channels may have a similar cross-section, each of the channels may be generally parallel with the first direction. The mesh structure may have a more random structure where the voids do not have an organised direction, consistent cross-section, or alignment. The width of the voids may advantageously be relatively small, about 0.5mm to 2mm of cross section, and numerous compared to the width of the flow channel.

When the device comprises a second pitot tube, the flow lineariser may further be configured to reduce turbulent fluid flow immediately upstream and/or immediately downstream of the second pitot tube when fluid flows in the second direction. The flow lineariser may advantageously reduce the turbulence generated by the second pitot tube.

The flow lineariser may comprise one or more elements extending from a position adjacent to the second pitot tube toward the distal end of the flow channel in a direction substantially parallel to the first direction. This may advantageously reduce turbulence downstream of the second pitot tube when fluid flows in the second direction.

Alternatively or additionally, the flow lineariser may comprise one or more elements extending from the second pitot tube toward the distal end of the flow channel in a direction substantially parallel to the first direction. This may advantageously reduce turbulence upstream of the second pitot tube when fluid flows in the second direction.

The one or more elements may comprise or consist of fins extending parallel to the first direction. Alternatively, the elements may comprise or consist of one or more tubes aligned parallel to the first direction. Alternatively or additionally, the elements may comprise or consist of a honeycomb structure.

When the flow lineariser comprises a conduit, the second port may be received in the conduit. The conduit may extend from the first pitot tube to the second pitot tube. The conduit may extend downstream of the second pitot tube. The conduit may extend upstream of the second pitot tube.

The flow lineariser may comprise at least a portion of an external surface of the second pitot tube. The portion of the surface of the second pitot tube that forms part of the flow lineariser may have similar features to the portion of the surface of the first pitot tube that forms of the flow lineariser described above.

The flow lineariser may comprise a mesh material surrounding the second port. By forcing the flow to pass through the voids and channels in a mesh material, vortices and other large swirling patterns in the flow can be aligned to smaller flow patterns, which more closely resemble a smooth or laminar flow pattern. The mesh structure may have a plurality of channels that align to be generally straight. Each of the channels may have a similar cross-section, each of the channels may be generally parallel with the first direction. The mesh structure may have a more random structure where the voids do not have an organised direction, consistent cross-section, or alignment. The width of the voids may advantageously be relatively small, about 0.5mm to 2mm of cross section, and numerous compared to the width of the flow channel.

The first pitot tube may be removably couplable to the device housing. This may facilitate cleaning of the first pitot tube separately from the device housing. In particular, the first pitot tube may be cleaned separately to the flow channel. It may be easier to clean the first pitot when it is not received in the flow channel because it may be easier to access the first pitot tube. Furthermore, it may be easier to clean the flow channel without the presence of the first pitot tube. For example, the device housing, and particularly the flow channel, may advantageously be immersed in an approved cleaning liquid after the first pitot tube has been removed, thus avoiding blockage of the pitot tube with the cleaning liquid. For example, the device housing, and particularly the flow channel, may be cleaned with an abrasive material or fluid jet after the first pitot tube has been removed, thus avoiding damage to the fine features of the first pitot tube that could invalidate the calibration.

When the device comprises a second pitot tube comprising the second port, the second pitot tube may be removably couplable to the device. Both the first and second pitot tube may be removably couplable to the device.

The device housing may comprise a first opening for receiving the first pitot tube. The device housing may comprise a second opening for receiving the second pitot tube.

The device may comprise a pitot tube module. The pitot tube module may comprise the first pitot tube and the second pitot tube. The pitot tube module may be removably couplable to the device housing. The pitot tube module may be removably couplable from the device housing as a single unit. This may advantageously simplify the removal and coupling of the first and second pitot tubes with the device housing.

Preferably, the pitot tube module may be integrally formed.

The device may comprise a seal between the pitot tube module and the device housing. The seal may be configured to seal an interface between the pitot tube module and the device housing when the pitot tube module is coupled to the device housing. This may advantageously reduce or eliminate fluid leakage out of the device housing. In particular, this may reduce or eliminate fluid leakage from the first and second opening of the device housing through the which the first and second pitot tubes may be received.

The seal may comprise or consist of an o-ring or a gasket.

The device may comprise a sensor module comprising the first and second sensor assemblies. The sensor module may be removably couplable to the device housing as a single unit. This may facilitate cleaning of the sensor module and the device housing.

When the device comprises a pitot tube module comprising the first pitot tube and the second pitot tube, the sensor module may be removably couplable to the pitot tube module as a single unit.

The first pitot tube preferably comprises a single first port. The single first port may preferably be aligned with the longitudinal axis of the flow channel.

Alternatively, the first pitot tube may comprise a plurality of first ports. Each of the first ports may face towards a direction of flow of fluid flowing through the flow channel when fluid flows from the proximal end to the distal end of the flow channel. Each of the first ports may face in the second direction. The plurality of first ports may be spaced apart along the length of the pitot the tube. At least one of the first ports may be located centrally within the flow channel.

Preferably, the device may comprise a single second port. The single second port may preferably be aligned with the longitudinal axis.

Alternatively, when the device comprises a second pitot tube, the second pitot tube may comprise a plurality of second ports. Each of the second ports may face towards a direction of flow of fluid flowing through the flow channel when fluid flows from the distal end to the proximal end of the flow channel. Each of the second ports may face in the first direction. The plurality of second ports may be spaced apart along the length of the pitot the tube. At least one of the second ports may be located centrally within the flow channel.

The device may comprise control circuitry configured to receive signals from the first and second sensor assemblies.

The device may be configured or configurable for performing a first fluid flow test. The control circuitry may be configured to measure or determine a pressure difference value during the first fluid flow test.

When the first and second sensor assembly comprise a differential sensor, the control circuitry may be configured to measure the pressure difference value based on signals received from the differential pressure sensor.

When the first and second sensor assembly each comprise a first sensor and a second sensor, the control circuitry may be configured to determine the pressure difference value. The control circuitry may be configured to measure the stagnation pressure based on signals from the first sensor of the respective first or second sensor assembly. The control circuitry may be configured to measure the static pressure based on signals received from the second sensor of the respective first or second sensor assembly while fluid flows in the first direction through the flow channel. The control circuitry may be configured to determine the dynamic pressure value by subtracting the measured static pressure from the measured stagnation pressure.

The control circuitry may be configured to output a result for the first flow test. The result may be based on the measured or determined dynamic pressure value.

The control circuitry may be configured such that the result of the first flow test is based on signals from one or both of the first and second sensor assemblies. The control circuitry may be configured to measure or determine a dynamic pressure value for the first flow test based on signals from one or both of the first and second sensor assemblies.

The control circuitry may be configured such that the result of the first flow test may be based on signals from only one of the first and second sensor assemblies. The control circuitry may be configured to measure or determine a dynamic pressure value for the first flow test based on signals from only one of the first and second sensor assemblies.

The control circuitry may be configured to output the result of the first fluid flow test based on signals from only the second sensor assembly. This may be the case when the first fluid test has a relatively low range of expected pressure, for example a lung inflammation test such as an FeNO test.

The control circuitry may be configured to output the result of the first fluid flow test based on signals from the first sensor assembly only. This may be the case when the first fluid test has a relatively high range of expected pressure, for example lung function test such as a spirometry test.

The control circuitry may be configured to select whether to base the result of the first fluid flow test on signals from the first sensor assembly or the second sensor assembly.

The control circuitry may be configured to select whether to base the result of the first fluid flow test on signals from the first sensor assembly or the second sensor assembly based on the flow test, and preferably the type of flow test, to be performed.

The control circuitry may be configured such that the selection comprises looking up the particular type of flow test to be performed in a database stored in a memory of the control circuitry and selecting signals from the first or second sensor assembly accordingly.

For example, it may be stored in the database that the second sensor assembly is suitable for performing a test measuring the concentration of a biomarker indicating lung inflammation, such as an FeNO test. If such a test is to be performed, the control circuitry may configured to select signals from the second sensor assembly. It may be stored in the database that the first sensor assembly is suitable for performing a lung function test such as a spirometry test. If such a test is to be performed, the control circuitry may configured to select signals from the first sensor assembly.

The control circuitry may be configured such that the selection may be based on determining if an initial pressure difference value falls outside a sensitive range of one of the sensor assemblies.

The control circuitry may be configured to operate on signals from both of the first and second sensors during the first fluid flow test in order to select the signals upon which the result to be output may be based. In this way, the signals from the first or second sensor assembly may be selected as being more appropriate or accurate for arriving at the result of the test without needing prior knowledge of the sensor assembly that is suitable for each test.

The control circuitry may be configured to initially measure or determine a pressure difference value during the first fluid flow test based on signals received from only one of the sensor assemblies. The control circuitry may be configured to determine if the initial pressure difference value falls outside of the sensitive range of the respective sensor assembly. The control circuitry may be configured such that, if the initial pressure difference for the respective sensor assembly does not fall outside of the sensitive range, a result based on signals from the respective sensor assembly is output. The control circuitry may be configured such that, if the initial pressure difference for the respective sensor assembly does fall outside of the sensitive range, a result based on signals from the other sensor assembly is output.

For example, the control circuitry may be configured to measure or determine an initial pressure difference value for the first flow test based on signals from the second sensor assembly. The control circuitry may be configured to compare the initial differential pressure value to a maximum threshold value for the sensitive range of the second sensor assembly. The control circuitry may be configured such that, if the initial differential pressure is less than the maximum threshold, a result based on signals from the second sensor assembly is output. The control circuitry may be configured such that, if the initial differential pressure is greater than or equal to the maximum threshold, a result based on signals from the first assembly is output instead.

The control circuitry may be configured to measure or determine an initial pressure difference value for the first flow test based on signals from the first sensor assembly. The control circuitry may be configured to compare the initial differential pressure value to a minimum threshold value for the sensitive range of the second sensor assembly. The control circuitry may be configured such that, if the initial differential pressure is greater than or equal to the minimum threshold, a result based on signals from the first sensor assembly is output. The control circuitry may be configured such that, if the initial differential pressure is less than the minimum threshold, a result based on signals from the second assembly is output instead.

Alternatively or additionally, the control circuitry may be configured to determine if an initial pressure difference value falls outside a sensitive range of one of the sensor assemblies by determining that the output of one of the first or second sensor assembly is in an error state. The control circuitry may be configured such that, if an error state is not determined, a result based on signals from the respective sensor assembly may be output. The controller circuitry may be configured such, if an error state is determined, a result based on signals from the other sensor assembly is output instead.

The control circuitry may be configured to detect an error state based on an error signal received from the respective sensor assembly. Alternatively, the control circuitry may be configured to determine an error state based on the output of the respective sensor assembly. For example, the control circuitry may be configured to determine an error state if the output of the respective initial sensor assembly is noisy or erratic.

The control circuitry may be configured to output a result for the first test based on signals received from both the first and second sensor assemblies received simultaneously. For example, the control circuitry may be configured to output a result that is based on an average of the pressure differential measured by the first sensor assembly and the pressure differential measured by the second sensor assembly.

It has been described how the control circuitry may be configured to output a result for the first test based on signals from one or both of the first and sensor assemblies. These signals may relate to single data points representing single measurements. However, the signals may also relate to a plurality of data points representing a plurality of measurements. The signals may relate to more than 1000, preferably more than 5000 data points.

The result for the first test may be based on a first portion of signals from the first sensor assembly and a second portion of signals from the second sensor assembly.

The device may be configured or configurable for performing a second fluid flow test. The control circuitry may be configured to output a result for the second flow test. The control circuitry may be configured to output a result for the second flow test that is based on the measured or determined dynamic pressure value.

As for the first fluid flow test, the control circuitry may be configured to output a result of the second flow test that is based on signals from one or both of the first and second sensor assemblies.

In one example, the control circuitry may be configured to output a result for the second fluid flow test that is based on signals from only one of the first and second sensor assemblies, as described in relation to the first flow test. Preferably, when the result of the first flow test is based on signals from one of the first and second sensor assemblies, the result of the second flow test may be based on signals from the other of the first and second sensor assemblies.

Similarly to the first fluid flow test, the control circuitry may be configured to select whether to base the result of the second fluid flow test on signals from the first sensor assembly or the second sensor assembly. This selection may be the same as described above in relation to the first test.

The control circuitry may be configured to output a result for the second fluid flow test based on signals received from both the first and second sensor assemblies simultaneously.

The control circuitry may comprise a microcontroller. The microcontroller may be configured to process the data received from the sensors. The processing of data may include performing smoothing to account for noise. The result of the processing of data may be used directly in test results, processed further to determine parameters such as inhale or exhale volume, or used to ensure that the breathing rate is within the requirements for the test. The control circuitry may instead be in communication with a portable computer comprising a microcontroller such as a laptop or smartphone. In these cases, the microcontroller of the portable device performs the data processing. The communication between the control circuitry and the portable device may be wireless a connection. The wireless connection may be a Bluetooth connection or a Wi-Fi connection.

The device may comprise a first configuration in which the device is configured to perform a first fluid flow test. The device may comprise a second configuration in which the device is configured to perform a second fluid flow test. Each of the tests that the device is configurable to perform may be a respiratory test.

In the second configuration of the device, the flow channel may be modified relative to the first configuration of the device. Between the first and second configuration, the flow channel may be modified by such that fluid in the flow channel is in fluid communication with different sensors and/or passes through different components of the device and/or so that properties of the fluid are changed. This may include increasing or reducing the flow resistance experienced by fluid in the flow channel, changing the cross-section of the flow channel, changing the temperature in the flow channel, adding or removing a flow lineariser, or providing additional inlets or outlets in the flow channel.

The device may comprise a primary component defining at least a portion of the flow channel and a first secondary component. The first secondary component may comprise a sensor that is not is part of the first and second sensor assemblies. The first secondary component may comprise a channel defined by a housing of the first second component. The step of modifying the flow channel may comprise engaging or disengaging the first secondary component to the primary component. When the first secondary component is engaged to the primary component the channel of the first secondary component may form at least a portion of the flow channel and/or the sensor of the first secondary component may be in fluidic communication with the flow channel. When the first secondary component is not engaged to the primary component the channel of the first secondary component may not form a portion of the flow channel and/or the sensor of the first secondary component may not be in fluidic communication with the flow channel.

In a first configuration of the device, device may be configured to perform a test for measuring the concentration of a biomarker indicating lung inflammation. The device may comprise a biomarker sensor for detecting the presence of the biomarker. In the first configuration of the device, the biomarker sensor may be in fluidic communication with the flow channel. Preferably, the biomarker sensor may be an electrochemical sensor. The biomarker sensor may not be part of the first or second sensor assemblies.

The device may comprise a biomarker sensing component which may comprise or consist of the biomarker sensor. The biomarker sensing component may be a first second component. In the first configuration of the device, the biomarker sensing component may be engaged to the device housing. The biomarker sensing component may be removably coupled to the device housing when the device is in the first configuration.

The biomarker sensing component may be suitable for performing an FeNO test. The biomarker may be nitric oxide concentration. The electrochemical sensor may be a sensor for detecting the presence of nitric oxide.

The control circuitry may be configured to operate on signals received from at least the second sensor assembly when the device is in the first configuration. This may be because a test for measuring the concentration of a biomarker indicating lung inflammation may typically be a relatively low flow test.

In a second configuration of the device, the device may be configured to perform a lung function test. The lung function test may be a spirometry test. In the second configuration of the device, the biomarker sensing component may not be engaged to the device housing. The biomarker sensor may not be in fluidic communication with the flow channel. In the second configuration of the device, there may be no secondary component engaged to the device housing. The flow channel may be defined only by the device housing and, if present, through a mouthpiece.

The control circuitry may be configured to operate on signals received from at least the first sensor assembly when the device is in the second configuration. This may be because a test for measuring lung function may typically be a relatively high flow test.

The device may comprise a mouthpiece connected to the proximal end or the distal end of the airflow channel. In use, a subject may inhale and/or exhale through the flow channel of the device via the mouthpiece. The mouthpiece may be removably coupled or couplable to the flow channel. In this way, the mouthpiece may be replaceable. For example, the mouthpiece may be replaced when the device is used by different subjects or between different testing periods using the device for a single subject.

The device may comprise a fluid outlet at the proximal end or distal end of the flow channel. When a mouthpiece is connected to one of the proximal end or distal end of the airflow channel, the fluid outlet may be at the other of the distal end or proximal end of the flow channel. Although the fluid outlet is referred to as outlet, fluid may flow into the fluid outlet.

In a second aspect there is provided a method of performing a fluid flow test using a device according to the first aspect.

The method comprises the step of: measuring or determining a difference between the pressure in the first pitot tube and the pressure at the second port using at least one of the first and second sensor assemblies.

When the device comprises a second pitot tube comprising the second port, the method may comprise measuring or determining the difference between the pressure in the first pitot tube and the pressure in the second pitot tube using at least one of the first and second sensor assemblies.

The difference between the pressure in the first pitot tube and the pressure at the second port (or in the second pitot tube, if present) may be the dynamic pressure. So, the above step of the method may comprise measuring or determining the dynamic pressure in the flow channel.

The method may comprise measuring or determining the dynamic pressure while fluid flows through the flow channel in the first direction. Preferably, the method may comprise measuring or determining the dynamic pressure while a subject inhales or exhales through the flow channel such that fluid flows in the first direction.

When the device comprises a second pitot tube comprising the second port and the second port faces in the first direction, the method may comprise measuring or determining the dynamic pressure when fluid flows through the flow channel in the first direction and then measuring or determining the dynamic pressure when fluid flows through the flow channel in the second direction, opposite to the first direction.

The first sensor assembly may comprise a differential pressure sensor. The differential pressure sensor of the first sensor assembly may be in fluidic communication with the first port and the second port. The second sensor assembly may comprise a differential pressure sensor. The differential pressure sensor of the second sensor assembly may be in fluidic communication with the first port and the second port.

The method may comprise measuring the dynamic pressure based on signals from the differential pressure sensor of at least one of the first and second sensor assemblies.

Alternatively, the first sensor assembly may comprise a first sensor configured to measure the pressure in the first pitot tube and a second sensor configured to measure the pressure at the second port (or in the second pitot tube, if present). The second sensor assembly may comprise a first sensor configured to measure the pressure in the first pitot tube and a second sensor configured to measure the pressure at the second port (or in the second pitot tube, if present).

The method may comprise measuring the stagnation pressure based on signals from the first sensor of at least one of the first and second sensor assemblies while fluid flows in the first direction through the flow channel. The method may further comprise measuring the static pressure using the second sensor of the respective first or second sensor assembly while fluid flows in the first direction through the flow channel.

The method may further comprise determining the dynamic pressure by subtracting the measured static pressure from the measured stagnation pressure.

The method may comprise performing a first fluid flow test using the device. The method may comprise outputting a result for the first flow test.

The result may be based on the measured or determined dynamic pressure value.

The device may comprise one or more additional sensors. For example, the one or more additional sensors may comprise at least one of an electrochemical sensor, a temperature sensor and a humidity sensor.

The step of calculating the result may alternatively or additionally comprise combining the measured or determined dynamic pressure value with one or more other values measured using the one or more additional sensors. For example, the first test may be an FeNO test. The result of the FeNO test may be a concentration of nitric oxide in the air which may be calculated based on a value representing nitric oxide levels measured by the electrochemical sensor as well as the measured or determined dynamic pressure. Furthermore, the result of the FeNO test may be calibrated using temperature and/or humidity values measured using the temperature and humidity sensors.

Alternatively, the result being based on the measured or determined dynamic pressure value may comprise using measured or determined dynamic pressure as an instruction for performing the respiratory test. For example, a biomarker test such as an FeNO test may require a user to exhale through the flow channel with a flow rate falling within a preferred range. The control circuitry may be configured to calculate a flow rate using the measured or determined dynamic pressure value. The flow rate may be an instantaneous flow rate. The control circuitry may be further configured to compare the calculated flow rate with the preferred range of flow rates which may be stored in a memory of the control circuitry. The device may be configured to provide a user with a first indication if the calculated flow rate falls within the preferred range. The device may be configured to provide a user with a second indication if the calculated flow rate falls outside of the preferred range. In this way, the user may advantageously be informed by the device whether the respiratory test is being performed correctly. The user may be directed to alter their breathing accordingly.

The result of the first flow test may be based on signals from one or both of the first and second sensor assemblies.

In one example, the result of the first flow test may be based on signals from only one of the first and second sensor assemblies.

The method may comprise outputting the result of the first fluid flow test based on signals from only the second sensor assembly. This may be the case when the first fluid test has a relatively low range of expected pressure, for example a lung inflammation test such as an FeNO test.

The method may comprise outputting the result of the first fluid flow test based on signals from the first sensor assembly only. This may be the case when the first fluid test has a relatively high range of expected pressure, for example a lung function test such as a spirometry test.

The method may comprise selecting whether to base the result of the first fluid flow test on signals from the first sensor assembly or the second sensor assembly.

The step of selecting may be based on the flow test, and preferably the type of flow test, being performed.

The selection may comprise looking up the particular type of flow test being performed in a database stored in a memory of the device and selecting signals from the first or second sensor assembly accordingly.

For example, it may be stored in the database that the second sensor assembly is suitable for performing a test measuring the concentration of a biomarker indicating lung inflammation, such as an FeNO test. It may be stored in the database that the first sensor assembly is suitable for performing a lung function test such as a spirometry test. By looking up the test to be performed in the database, the appropriate sensor assembly for the flow test to be performed may be selected.

The selection may be based on determining if an initial pressure difference value falls outside a sensitive range of one of the sensor assemblies.

Preferably, the method may comprise operating on signals from both of the first and second sensors during the first fluid flow test. However, the step of outputting the result may be based on the signals from only one of the first and second sensor assemblies. In this way, the signals from the first or second sensor assembly may be selected as being the most appropriate or accurate for measuring or determining the output of the test without needing prior knowledge of the sensor assembly that is suitable for each test.

The method may comprise initially measuring or determining a pressure difference value during the first fluid flow test based on signals received from only one of the sensor assemblies. The method may further comprise determining if the initial pressure difference value falls outside of the sensitive range of the respective sensor assembly. If the initial pressure difference for the respective sensor assembly does not fall outside of the sensitive range, then the method may comprise outputting a result based on signals from the respective sensor assembly. If the initial pressure difference for the respective sensor assembly does fall outside of the sensitive range, then the method may comprise outputting a result based on signals from the other sensor assembly.

For example, the method may comprise measuring or determining an initial pressure difference value for the first flow test based on signals from the second sensor assembly. The method may comprise comparing the initial differential pressure value to a maximum threshold value for the sensitive range of the second sensor assembly. If, during the first fluid test, the initial differential pressure is less than the maximum threshold, then the method may comprise outputting a result based on signals from the second sensor assembly. If, during the first fluid test, the initial differential pressure is greater than or equal to the maximum threshold, then the method may comprise outputting a result based on signals from the first assembly instead.

The method may comprise measuring or determining an initial pressure difference value for the first flow test based on signals from the first sensor assembly. The method may comprise comparing the initial differential pressure value to a minimum threshold value for the sensitive range of the second sensor assembly. If, during the first fluid test, the initial differential pressure is greater than or equal to the minimum threshold, then the method may comprise outputting a result based on signals from the first sensor assembly. If, during the first fluid test, the initial differential pressure less than the minimum threshold, then the method may comprise outputting a result based on signals from the second assembly instead.

Alternatively or additionally, the step of determining if an initial pressure difference value falls outside a sensitive range of one of the sensor assemblies may comprise determining that the output of respective first or second sensor assembly is in an error state. If an error state is not determined, the result may be based on signals from the respective initial sensor assembly. If an error state is determined, the result may be based on signals from the other sensor assembly.

An error state may be detected based on an error signal received from the respective sensor assembly. Alternatively, an error state may be determined based on the output of the respective sensor assembly. For example, the control circuitry may be configured to determine an error state if the output of the respective initial sensor assembly is noisy or erratic.

The method may comprise outputting a result for the first flow fluid test based on signals received from both the first and second sensor assemblies received simultaneously. For example, the method may comprise outputting a result that is an average of the pressure differential measured by the first sensor assembly and the pressure differential measured by the second sensor assembly.

It has been described how the control circuitry may be configured to output a result for the first test based on signals from one or both of the first and sensor assemblies. These signals may relate to single data points representing single measurements. However, the signals may also relate to a plurality of data points representing a plurality of measurements. The signals may relate to more than 1000, preferably more than 5000 data points.

The result for the first test may be based on a first portion of signals from the first sensor assembly and a second portion of signals from the second sensor assembly.

The method may additionally comprise performing a second fluid flow test using the device. The method may comprise outputting a result for the second flow test. The method may comprise outputting the measured or determined dynamic pressure value as the result of the second fluid flow test. Alternatively or additionally, the method may comprise calibrating the measured or determined dynamic pressure value as the result of the second fluid flow test.

Like the first fluid flow test, the result of the second fluid flow test may be based on signals from one or both of the first and second sensor assemblies.

In one example, the result of the second fluid flow test may be based on signals from only one of the first and second sensor assemblies. Preferably, when the result of the first fluid flow test is based on signals from one of the first and second sensor assemblies, the result of the second flow test may be based on signals from the other of the first and second sensor assemblies.

Similarly to the first fluid flow test, the method may comprise selecting whether to base the result of the second fluid flow test on signals from the first sensor assembly or the second sensor assembly. This selection may be the same as described above in relation to the first test.

The method may comprise outputting a result for the second test based on signals received from both the first and second sensor assemblies received simultaneously.

The device may comprise a first configuration in which the device is configured to perform a first fluid test. The device may comprise a second configuration in which the device is configured to perform a second fluid flow test. The method may comprise placing the device in one of these configurations prior to performing the respective fluid flow test.

The method may further comprise reconfiguring the device between the step of performing a first flow test and the step of performing a second fluid flow test.

The step of reconfiguring the device to perform a fluid flow test may comprise modifying the flow channel. The step of modifying the flow channel may comprise modifying the flow channel such that fluid is in fluid communication with different sensors and/or passes through different components of the device and/or so that properties of the fluid are changed. This may include increasing or reducing the flow resistance experienced by fluid in the flow channel, changing the cross-section of the flow channel, changing the temperature in the flow channel, adding or removing a flow lineariser, or providing additional inlets or outlets in the flow channel.

The device may comprise a primary component defining at least a portion of the flow channel and a first secondary component. The first secondary component may comprise a sensor that is not is part of the first and second sensor assemblies. The first secondary component may comprise a channel defined by a housing of the first second component. The step of modifying the flow channel may comprise engaging or disengaging the first secondary component to the primary component. When the first secondary component is engaged to the primary component the channel of the first secondary component may form at least a portion of the flow channel and/or the sensor of the first secondary component may be in fluidic communication with the flow channel. When the first secondary component is not engaged to the primary component the channel of the first secondary component may not form a portion of the flow channel and/or the sensor of the first secondary component may not be in fluidic communication with the flow channel.

The first secondary component may have a first position relative to the primary component in the first configuration and a second position relative to the primary component in the second configuration. The device may comprise a plurality of secondary components, with each of the secondary components being designed for a specific respiratory test.

The device may comprise a biomarker sensing component for performing a test for measuring the concentration of a biomarker indicating lung inflammation. The biomarker sensing component may be one of the secondary components. The biomarker sensing component may comprise or consist of an electrochemical sensor. The electrochemical sensor may be in fluidic communication with the channel of the biomarker sensing component.

The step of configuring the device to perform the test for measuring the concentration of a biomarker indicating lung inflammation may comprise engaging the biomarker sensing component with the primary component. This may comprise connecting the biomarker sensing component to the primary component.

Features described in relation to one aspect may be applied to other aspect of the invention. In particular features and advantages of those features described in relation to the device of the first aspect may be applied to method of the second aspect, and vice versa.

### Brief Description of the Drawings

Embodiments in accordance with the invention will now be described, by way of example only, with reference to the accompanying drawings, in which:
Figure 1 is a horizontal cross-sectional view of a device according to a first embodiment of the invention, the device in a first configuration for performing a first respiratory test;
Figure 2 is a horizontal cross-sectional view of a portion of the device of Figure 1 in the first configuration;
Figure 3 is a vertical cross-section of the device of Figure 1, the cross-section being defined between a first and second pitot tube of the device of Figure 1;
Figure 4 is an exploded perspective view of a device housing portion, a pitot tube module and a sensing assembly module of the device of Figure 1;
Figure 5 is an exploded horizontal cross-sectional view corresponding to the view of Figure 4;
Figure 6 is a horizontal cross-sectional view of the device of Figure 1 in a second configuration for performing a second respiratory test in which a biomarker sensing module is engaged to the device housing;
Figure 7 is flow chart showing a method of using the device of the first embodiment;
Figure 8 is a flow chart showing a method of selecting which of a first and second differential pressure sensor of device 100 to use in the method of Figure 8;
Figure 9 is a horizontal cross-sectional view of a second embodiment of the pitot tube module; and
Figure 10 is a is horizontal cross-sectional view of a device according to a second embodiment of the invention, the device in a first configuration for performing a first respiratory test.

### Detailed Description

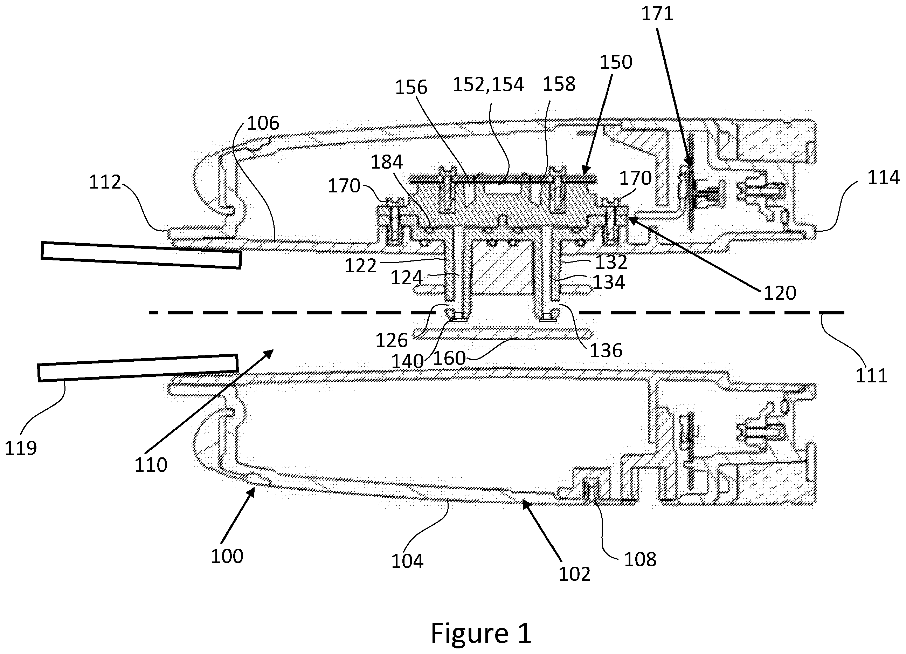

Figure 1 is a horizontal cross-sectional view of a device 100 according to a first embodiment of the invention. The device 100 is in a first configuration for performing a first respiratory test.

The device 100 comprises a primary component 102. The primary component 102 comprises an outer housing 104 and an inner housing 106.

The outer housing 104 acts as a casing for the inner housing 106 and the other components of the primary component 102. The outer housing 104 is removable from the inner housing 106 by unscrewing a screw 108 and sliding the outer housing 104 longitudinally with respect to the inner housing 106.

The inner housing 106 defines an airflow channel 110. The airflow channel 110 has a proximal end 112 and a distal end 114. The airflow channel 110 extends longitudinally and is axis symmetric about a central axis 111 represented by the broken line in Figure 1. The airflow channel 110 has a circular cross-section along the central axis.

A mouthpiece 119 is removably coupled to the primary component 102 such that the mouthpiece 119 is in fluidic communication with the airflow channel 110 at the proximal end 112 of the airflow channel 110. The mouthpiece being removably coupled to the primary component 102 means that the mouthpiece is replaceable. For example, if the device 100 is used by multiple subjects, the mouthpiece can be replaced for each subject.

A subject using the device can exhale or inhale through the mouthpiece 119 to draw air through the airflow channel 110. When a user exhales through the mouthpiece, air flows through the airflow channel 110 in a first direction such that air flows from the proximal end 112 to the distal end 114. The first direction is parallel to the central axis 111. When a user inhales through the mouthpiece, air flows through the airflow channel 110 in a second direction opposite to the first direction such that air flows from the distal end 114 to the proximal end 112. The second direction is parallel to the central axis 111.

The inner housing 106 is shaped such that the airflow channel 110 tapers slightly such that the diameter of airflow channel 110 decreases slightly between the proximal end 112 and a central point between the proximal end 112 and the distal end 114. The tapering of the airflow channel 110 is symmetrical about the central point. This shape of inner housing 106 allows for efficient injection moulding of the inner housing 106.

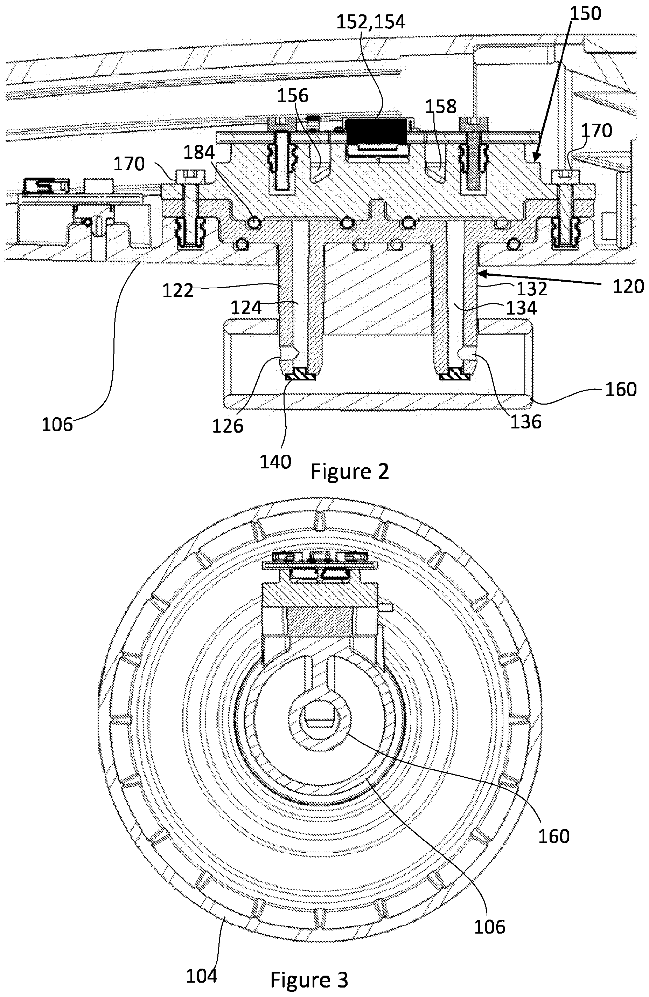

The primary component 102 comprises a pitot tube module 120 and a sensor module 150. Figure 2 is a horizontal cross-sectional view of a portion of the device of Figure 1 centred on the pitot tube module 120 and the sensor module 150.

The pitot tube module 120 comprises a first pitot tube 122 and a second pitot tube 132.

The first pitot tube 122 comprises a first pitot tube housing that defines an internal passage 124 that extends perpendicularly to the central axis 111. The first pitot tube housing also defines an aperture that extends through the housing to meet the internal passage 124. The aperture forms a first air inlet or first port 126. The first port 126 is in fluidic communication with the airflow channel 110 and so the internal passage 124 of the first pitot tube 122 is in fluidic communication with the airflow channel 110 via the first port 126. The first port 126 is aligned with the central axis 111. The device 100 of the first embodiment comprises a single first port 126.

The second pitot tube 132 similarly comprises a second pitot tube housing that defines an internal passage 134 that extends perpendicularly to the central axis 111. The first pitot tube housing also defines an aperture that extends through the housing to meet the internal passage 134. The aperture forms a second air inlet or second port 136. The second port 136 is in fluidic communication with the airflow channel 110 and so the internal passage 134 of the second pitot tube 132 is in fluidic communication with the airflow channel 110 via the second port 136. The second port 136 is aligned with the central axis 111. The device 100 of the first embodiment comprises a single second port 136.

The first port 126 faces in a second direction. The first port 126 therefore faces air flowing through the airflow channel 110 towards the first port 126 when air flows in the first direction (i.e. when a subject exhales through the device 100). The first port 126 faces towards the proximal end 112 of the airflow channel 110. The first port 126 lies in a plane, the normal of which is parallel to the central axis 111.

The second port 136 faces in the first direction. The second port 136 therefore faces away from the second direction. The second port 136 therefore faces away from air flowing through the airflow channel 110 when air flows in the first direction (i.e. when a subject exhales through the device 100). The second port 136 faces in an opposite direction to the first port 126. The second port 136 faces towards the distal end 114 of the airflow channel 110. The second port 136 lies in a plane, the normal of which is parallel to the central axis 111.

The sensor module 150 comprises a first differential pressure sensor 152 and a second differential pressure sensor 154. These sensors are shown as a single block in the figures. The first differential pressure sensor 152 can measure differential pressures of up to ±1500 Pa. The second differential pressure sensor 154 can measure differential pressures of up to ±125 Pa. Both differential pressure sensors are provided by the same manufacturer and from the same family of sensors produced by that manufacturer. This ensures each sensor has a similar response to factors like temperature, humidity, altitude, and aging. For example, the first differential pressure sensor may be a Sensirion SDP33 digital differential pressure sensor. The second differential pressure sensor may be a Sensirion SDP32 digital differential pressure sensor. Both the Sensirion SDP33 digital differential pressure sensor and the Sensirion SDP32 digital differential pressure sensor are available from Allied Enterprises, 26021 Center Ridge Road, Westlake, OH 44145, USA for example.

The sensor module 150 comprises a first chamber 156 and a second chamber 158. The first chamber 156 is in fluidic communication with the internal passage 124 of the first pitot tube 122. The second chamber 158 is in fluidic communication with the internal passage 134 second pitot tube 132.

The first differential pressure sensor 152 comprises a first port in fluidic communication with the first chamber 156 and a second port in fluidic communication with the second chamber 158. The first differential pressure sensor 152 is configured to measure the difference in pressure between the first and second ports of the first differential pressure sensor 152. So, the first differential pressure sensor 152 is configured to measure the difference in pressure between the first and second chambers 156,158 of the sensor module 150. By measuring the difference in pressure between the first and second chambers 156,158, the first differential pressure sensor 152 is also configured to measure the difference in pressure between the internal passages 124,134 of the first and second pitot tubes.

A subject can exhale through the mouthpiece 119 such that air flow through the airflow channel in the first direction. Because the first port 126 faces air flowing in the first direction, the first port 126 is exposed to the stagnation pressure created by a user exhaling through the mouthpiece 119. Because the second port 136 faces away from the second direction, the second port 136 is exposed to only the static pressure of air flowing in the first direction through the air flow channel 110. By measuring the difference in pressure between the first chamber 156 and the second chamber 158, the first differential pressure sensor is configured to measure the dynamic pressure of air flowing in the airflow channel 110 in the first direction.

If, instead of exhaling, a subject inhales through the mouthpiece 119, then air will flow through the airflow channel 110 in the second direction. The second port 136 faces the first direction and the first port 126 faces the second direction. So, when a subject inhales, it is the second port 136 that is exposed to the stagnation pressure created by air flowing in the second direction and it is the first port 126 that is exposed to only the static pressure. By measuring the difference in pressure between the first chamber 156 and the second chamber 158, the first differential pressure sensor is again configured to measure the dynamic pressure of air flowing in the airflow channel 110 but this time in the second direction.

So, the opposing arrangement of the first and second pitot tubes 122,132 means that the dynamic pressure of air flowing the airflow channel 110 can be measured using a single sensor (the first differential pressure sensor 152) when air flows in either the first direction or the second direction. Like the first differential pressure sensor 152, the second differential pressure sensor 154 comprises a first port in fluidic communication with the first chamber 156 and a second port in fluidic communication with the second chamber 158. So, like the first differential pressure sensor 152, the second differential pressure sensor 154 is configured to measure the dynamic pressure of air flowing in the airflow channel 110 in either first or second direction. As above, the sensitive range of the first differential pressure sensor 152 is different to that of the second differential pressure sensor 154 are different. During use of the device, measurements from one of the sensors can be selected as being most appropriate depending on the test being performed and/or the test conditions. Alternatively, measurements from both of the first and second differential pressures sensors can be combined. This is described in more detail below.

In some embodiments, the first differential pressure sensor 152, is replaced by a first pair of sensors and the second differential pressure sensor 154 is replaced by a second of sensors. The first pair of sensors is sensitive to a higher range of pressure than the second pair of sensors. One of the sensors in each pair of sensors is configured to measure the pressure of the first chamber 156. The other of the sensors in each pair of sensors is configured to measure the pressure in the second chamber 158. So, each pair of sensors measures both the static pressure and the dynamic pressure. The device comprises control circuitry to determine the difference between the pressure measured by the different sensors to determine the dynamic pressure.

The first and second pitot tubes 122,132 are symmetrical to one another. In this way, each of the first and second pitot tubes 122,132 will respond to pressure within the airflow channel in a corresponding way regardless of the direction of airflow.

The internal passages 124, 134 of the first and second pitot tubes 122,132 extend perpendicularly along the full length of the pitot tubes. This is an artefact of the manufacturing process of the first and second pitot tubes 122,132. In particular, the pitot tubes are manufactured rapidly and cheaply using injection moulding. However, because the internal passages 124,134 extend along the full length of the pitot tubes, there is an opening at a bottom end of each of the pitot tubes that is closest to the central axis 111. In the embodiment of Figure 1, optional caps 140 are provided to close the bottom end of the first and second pitot tubes 122,132. The caps 140 reduce the noise in the measured pressures.

The device 100 comprises a flow lineariser in the form of conduit 160 defined by the device housing. The purpose of the flow lineariser is to reduce turbulent airflow immediately upstream and immediately downstream of the first and second pitot tubes 122,132 when fluid flows in either the first or the second direction.

The conduit 160 is tubular and is positioned within the airflow channel 110. A longitudinal axis of the conduit 160 extends parallel to and is aligned with the central axis 111. The first port 126 of the first pitot tube 122 and the second port 136 of the second pitot tube 132 are both received in the conduit 160.

When air flows in the first direction, the air will pass the first pitot tube 122 and then pass the second pitot tube 132. As the air passes the first pitot tube 122, turbulence can be created. The presence of the conduit 160 substantially reduces this turbulence before the air reaches the second port 136. Similarly, the presence of the conduit 160 substantially reduces turbulence created by air passing the second pitot tube 132 before the air reaches the first port 126 when air flows in the second direction. Turbulent airflow can create noise in the measured pressures. So, by reducing turbulent airflow, the conduit 160 reduces noise in the pressure measurements made by the first and second differential pressure sensors 152,154.

The conduit also substantially reduces turbulence in the air flowing in the airflow channel that is not caused by the air passing over the first and second pitot tubes 122,132.

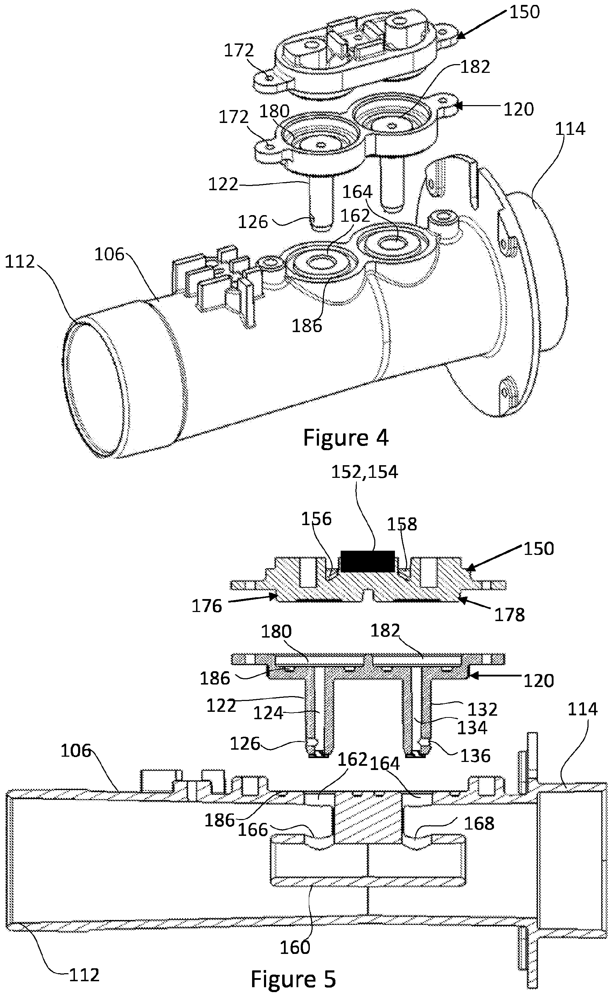

The pitot tube module 120 is removably coupled to the device housing 100. The sensor module 150 is removably coupled to the pitot tube module 120. This is shown more clearly in Figures 3 and 4 which are, respectively, perspective and horizontal cross-sectional views showing the device housing, the pitot tube module 120 and the sensor module 150 separately from the rest of the device 100 and exploded.

The pitot tube module 120 is integrally formed with the first and second pitot tubes 126,136 being connected to one another. The device housing comprises first and second apertures 162,164 through which the first and second pitot tubes 122,132 are received when the pitot tube module 120 is coupled to the device housing. The conduit 160 also comprises first and second apertures 166,168 through which the first and second pitot tubes 122,132 are received when the pitot tube module 120 is coupled to the device housing.

The sensor module 150 comprises a first portion 176 in which the first chamber is formed and a second portion 178 in which the second chamber is formed. When the sensor module 150 is coupled to the pitot tube module 120, the first portion 176 is received in a first cavity 180 of the pitot tube module 120 and the second portion 178 is received in a second cavity 182 of the pitot tube module 120.

To maintain pressure in the flow channel and avoid flow unintentionally bypassing the first and second differential pressure sensors 152,154, seals are provided at interfaces of the pitot tube module 120 with the device housing and the sensor module 150 with the pitot tube module 120. The seals are in the form of o-rings 184 received in channels 186 in the pitot tube module 120 and the sensor module 150. The o-rings are visible in Figures 1 and 2. The channels 186 are most clearly shown in Figure 4 and 5.

Screws 170 (visible in Figure 1 and 2) are used to secure the pitot tube module 120 to the sensor module 150 and the pitot tube module 120 to the device housing. The screws 170 pass through holes 172 on the pitot tube module 120 and the sensor module 150 and are attached to fixings in the device housing. In order to remove the pitot tube module 120 and the sensor module 150 from the device housing, the outer housing 104 is first removed. Screws 170 are then unscrewed.

As the pitot tube module 120 can be removed, this module can be cleaned more easily by a method such as immersing it in an approved cleaning liquid. All the internal flow paths are straight, which has benefits both for simplifying manufacture (e.g. by injection moulding) as well as enabling easier cleaning. Difficult to access geometry, where there is more likely to be bacteria build-up, is prevented. An upstream filter (e.g. using a mouthpiece) may be used in addition to the modular components with straight internal flow paths to minimize the risk of blockages in the pitot assembly.

The device 100 further comprises control circuitry 171. The control circuitry 171 is configured to receive signals from the first and second differential sensors 152,154. Based on those signals, the control circuitry 171 is configured to measure the dynamic pressure during a test.

The device 100 as shown in Figure 1 is in a first configuration. In this first configuration, the device 100 is configured for performing a lung function test, in a particular a spirometry test. Spirometry tests are used to indicate mechanical properties of the airways of the subject. The flow rate is measured and can be integrated over time to calculate lung volumes. The maximum exhaled flow rate is an indicator of airway resistance and obstruction; resistance to flow increases as airway diameter decreases. Obstructed or narrowed airways suggest the subject may have a respiratory condition such as asthma or COPD.

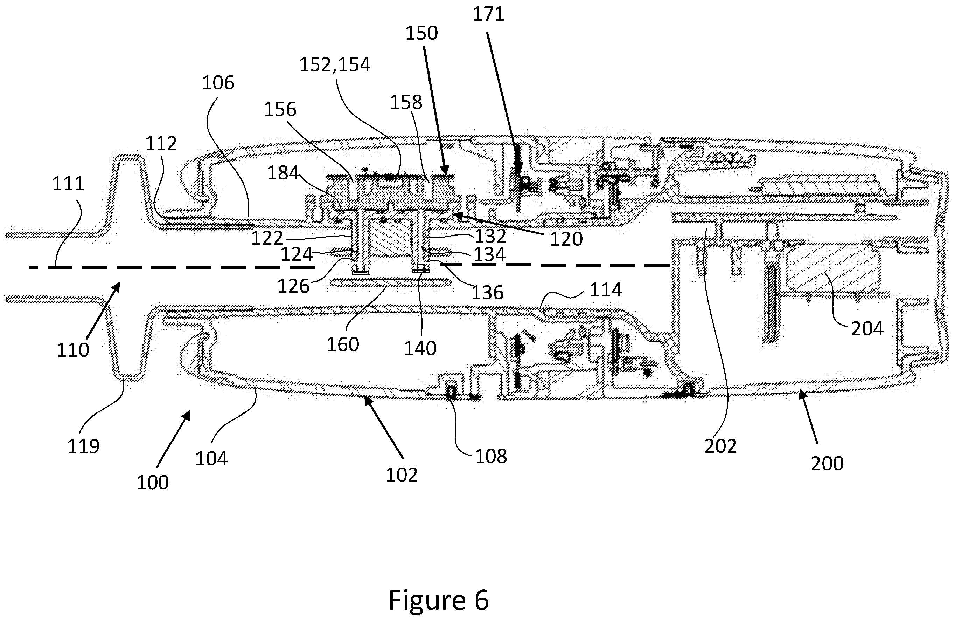

The device 100 can be reconfigured into a second configuration, as shown in Figure 6. In the second configuration, the device 100 is configured for performing a test for measuring the concentration of a biomarker indicated lung inflammation. **In** particular, in the second configuration, the device 100 is configured for performing an FeNO test wherein the biomarker is nitric oxide.

Nitric oxide concentration is used to indicate biological processes such as eosinophil activity. Eosinophil activity can be raised as part of the airway inflammation process. High exhaled nitric oxide concentration is used as an indicator for the subtype of a respiratory disease and probable response to inhaled corticosteroids. Exhaled nitric oxide concentration can be monitored over time to determine response to a treatment, anticipate attacks or exacerbations or periods of severe symptoms, determine if a patient is taking their prescribed treatment regularly and with good technique, and to titrate dosage of a treatment to a level that controls the patient's condition but without excess that is wasteful and can cause side effects.

Relative to the first configuration, the device 100 in the second configuration has been modified such that a secondary component 200 has been engaged to the primary component 102.

The secondary component 200 is nitric oxide sensing component. The secondary component 200 defines an airflow channel portion 202 that is in fluidic communication with the airflow channel 110 of the primary component 102. At the bottom of the secondary component 202 is a nitric oxide sensor 204. The nitric oxide sensor 204 is an electrochemical sensor and is an AlphaSense NO-B4 which is available from Sensor Technology House 300 Avenue West, Skyline 120, Great Notley, Braintree CM77 7AA. The sensor contains an electronics board that establishes electrode voltages for electrochemical reactions within the sensor and amplifies the signal produced by the sensor. The nitric oxide sensor 204 is in fluidic communication with the airflow channel portion 202. So, the concentration of nitric oxide in air flowing through the air flow channel portion 202 is measured by the nitric oxide sensor 204.

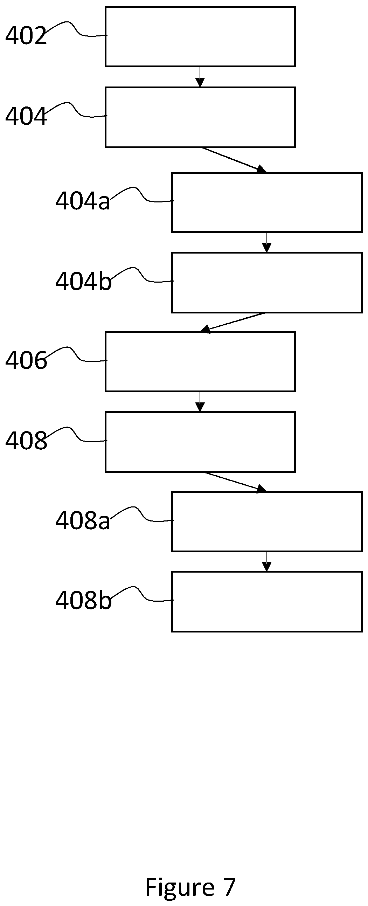

Figure 7 is a flow chart representing a method of using the device 100 to perform a plurality of respiratory tests.

Step 402 of the method is to configure the device 100 in the first configuration. If there are any secondary components engaged to the primary component 102 prior to performing step 402, then step 402 comprises disengaging the second component.

Step 404 of the method is to perform a spirometry test while the device 100 is in the first configuration. During the spirometry test, a subject will exhale through the mouthpiece 119 with a relatively high flow rate of up to 850 litres per minute.

Step 404 of the method comprises step 404a of selecting which of the first and/or second pressure differential pressure sensors 156,158 to base a result for the spirometry test on.

Step 404 of the method further comprises step 404b of outputting a result for the spirometry test which is based on a dynamic pressure value measured using the selected first and/or second pressure differential pressure sensor 156,158 of step 404a.

The result that is output in step 404b is a metric that is calculated using a flow rate which in turn is calculated using the measured dynamic pressure value. The measured dynamic pressure value is calibrated to calculate the flow rate.

When the device is to output flow rates, calibration coefficients are applied to the dynamic pressure value to calculate the equivalent flow rates.

In some examples, the calibration coefficients are pre-stored in a memory of the control circuitry 171.

In other examples, the calibration coefficients are determined. In order to determine the calibration coefficients, the device is put in series with a trusted and calibrated flow sensor. The distance and pressure drop between the trusted flow sensor and the pitot flow measurement device should be low.

Flow is provided through the device at a series of flow rates. The time series of data points from the trusted flow sensor (units: litres per second) and a differential pressure sensor (units: Pascals) is aligned in time with the time series of data points. The timeseries pairs of data points are correlated through a calibration regression.

This procedure is completed for all differential pressure sensors in the pitot flow measurement device. The procedure is completed for flow in both directions in the flow channel.

Preferably, the correlation of the calibration equation is forced so that the point at zero flow is the same for both flow directions. For example, the zero flow for the inhale and exhale flow directions could be forced in the calibration equation to be zero Pascals.

The calibration coefficients are saved into a database of the control circuitry 171 of the device.

Each metric gives an indication of the mechanical properties of the patient's lungs.

Examples of metrics include Peak Expiratory Flow (PEF), Forced Expiratory Volume in 1 second (FEV1), Forced Expiratory Flow (FEF) and Force Expiratory Volume (FEV) and finally Force Inspiratory Flow (FIF). Lung volumes are also calculated by integrating flow rate over time.

To measure PEF the subject inflates their lungs to a maximum volume, then exhales with as high a flow rate as they are able to produce. The PEF value is the maximum flow rate produced.

To measure FEV1 the subject inflates their lungs to a maximum volume, then exhales with as high a flow rate as they are able to produce. The FEV1 value is the maximum expired volume within the first second of expiration, typically measured in litres.

FEF and FEV are similar to PEF and FEV1 respectively but are more general metrics representing any expiration flow rate rather than peak flow rate as well volumes over any time interval rather than a fixed first second of expiration. They can be measured at any point of the exhalation. FIF, similar to FEF but for inhalation rather than exhalation, is also calculated.

The tidal volume (VT) is calculated as the volume of gas exchanged in one breath of normal tidal breathing. Tidal breathing is defined as inhalation and exhalation during restful breathing. At rest, also known as "quiet breathing", tidal breathing tends to have a narrow range between the depth of the breath and amount that is exhaled and tends to be fairly consistent. Respiratory diseases can change the normal character of this breathing and cause inconsistencies over a number of breaths.

The Forced Vital Capacity (FVC) can also be calculated, this is the volume exchanged from a maximum inhalation and maximum exhalation. Other volume measurements that are routinely used in clinical assessment and research can also be calculated using the device, most are calculated as the extreme inspiration or expiration volume relative to a minimum or maximum volume from tidal breathing.

Step 406 of the method is to configure the device 100 in the second configuration. If there are any secondary components engaged to the primary component 102 other than the nitric oxide sensing component 200, then step 406 comprises disengaging those secondary components. Step 406 comprises engaging the nitric oxide sensing component 200 to the primary component 102.

Step 408 of the method is to perform an FeNO test while the device 100 is in the second configuration. During the FeNO test, a subject will exhale through the mouthpiece 119 with a relatively low flow rate of about 3 litres per minute for about 10 seconds or 12 seconds for adults or about 6 seconds for young children. The intention of this test is to measure nitric oxide that is produced from the distal airways. Distal airways are airways with a diameter of less than 2 mm. These airways contribute to gas exchange in the lungs and cause only a small amount of resistance to air passing through them. Distal airways encourage laminar flow of air. Exhalation should be against a positive expiratory pressure, caused by an increased resistance to the exhaled flow in the device. The positive expiratory pressure ensures the nasal velum remains shut. The expiratory pressure should be at least 5 cm H₂O to keep the velum shut but pressures above 20 cm H₂O can be uncomfortable for the subject. Air in the nasal passages can have high concentrations of nitric oxide, which can contaminate the exhaled air from the lungs if the velum opens during the exhalation.

Step 408 of the method comprises step 408a of selecting which of the first and/or second pressure differential pressure sensors 156,158 to base a result for the FeNO test on.

Step 408 of the method further comprises step 408b of outputting a result for the FeNO test which is based on a dynamic pressure value measured using the selected first and/or second pressure differential pressure sensor 156,158 of step 408a.

The result for the FeNO test that is output at step 408b is a value for the nitric oxide concentration. A value for nitric oxide concentration is measured using the nitric oxide sensor 204. However, the nitric oxide sensor 204 is an electrochemical sensor.

The value measured by an electrochemical sensors is highly dependent on humidity and temperature. In some embodiments, the primary component 102 or the nitric oxide sensing component 200 comprises a humidity sensor configured to measure the humidity sensor and/or temperature sensor configured to measure the humidity or temperature, respectively, of the exhaled air. In such cases, the value that is measured by the nitric oxide sensor 204 can also be adjusted in view of the measured humidity and/or temperature to more closely reflect the true nitric oxide concentration.

Steps 402 and 404 of the method are performed sequentially with respect to one another. Steps 406 and 408 of the method are performed sequentially with respect to one another.

Steps 402 and 404 of the method can be performed before or after steps 406 and 408. In other words, the device can be used to perform the spirometry test and the FeNO test in any order but it is preferred that the FeNO test is performed first and the spirometry test is performed last. In some embodiments, the method can comprise performed only one of the tests (e.g. only steps 402 to 404 or steps 406 to 408 of the method may be performed).

At least steps 404a, 404b, 408a and 408b are performed by the control circuitry 171 of the device 100. Each respective step a is performed before the respective step b.

Steps 404a and 408a of the method each comprise the step of selecting which of the first and/or second pressure differential pressure sensors 152,154 to base a result for the respective respiratory test on.

In a first embodiment, the selection is based on the type of flow test being performed. The control circuitry 171 of the device 100 comprises a memory in which a database is stored. The database stores that for spirometry tests the first differential pressure sensor 152 should be selected and that for FeNO tests the second differential pressure sensor 154 should be selected.

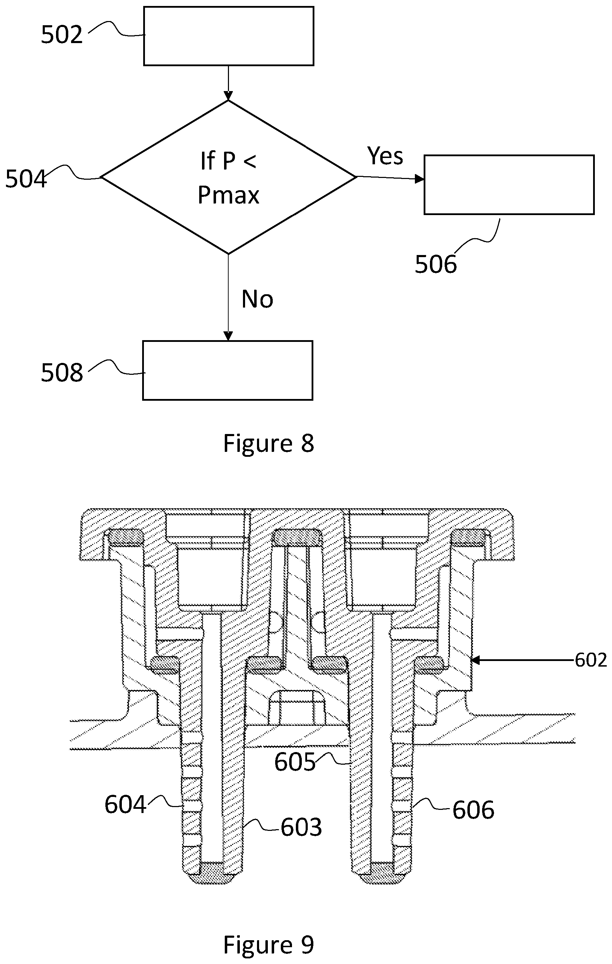

A second embodiment of the selection is shown in Figure 8 which is a flow diagram. In the second embodiment, the method comprises operating on signals from both of the first and second sensors during a fluid flow test in order to select the signals upon which the result to be output is based.

At step 502 of Figure 8, an initial pressure difference value is measured while a flow test is being performed based on signals from the second differential pressure sensor 154.

At step 504, the initial pressure difference value (P) is compared to a maximum threshold value (Pmax). Pmax represents the maximum value for the sensitive range of the second differential pressure sensor 154.

If the initial pressure difference value is less than the maximum threshold value then it can be determined that the second differential pressure sensor 154 is suitable for performing the test that the device 100 is configured to perform. So, step 506 of the method is performed in which a result based on signals from the second differential pressure sensor 154 is output.

If the initial pressure difference value is greater than or equal to the maximum threshold value, then it can be determined that the second differential pressure sensor 154 is not suitable for performing the test that the device 100 is configured to perform. So, step 508 of the method is performed in which a result based on signals from the first differential pressure sensor 152 is output instead.

Figure 9 is a horizontal cross-sectional view of a second embodiment of the pitot tube module 602 coupled to a portion of the device housing. The pitot tube module 602 of the second embodiment is similar to the pitot tube module 120 of the first embodiment and interacts similarly with a device housing 106 and a sensor module 150 as shown in Figure 1. The difference between the pitot tube module 602 of the first embodiment and the second embodiment is that the pitot tube module 602 comprises a first pitot tube 603 comprising a plurality of first ports 604 facing towards air flowing in the first direction and a second pitot tube 605 comprising a plurality of second ports 606 facing away from air flowing in the first direction. The plurality of first ports face 604 in the second direction. The plurality of second ports 606 face in the first direction. The plurality of first ports 604 are spaced apart along the length of the first pitot tube 603. The plurality of second ports 606 are spaced apart along the length of the second pitot tube 605.

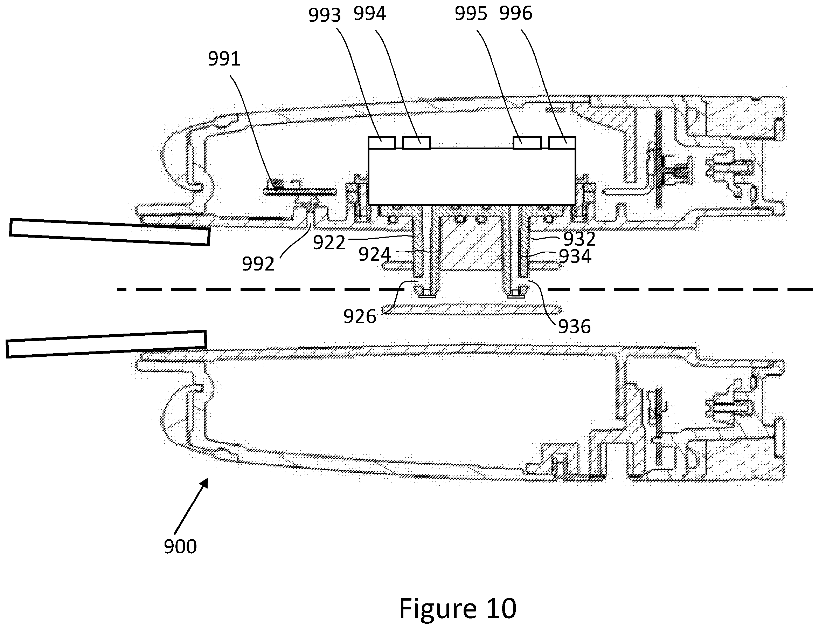

Figure 10 is a horizontal cross-sectional view of a device 900 according to a second embodiment of the invention.

The device 900 is in a first configuration for performing a first respiratory test.

The device 900 is similar to the device 100 of Figure 1, as are the methods of operation, and so shall be described only with respect to the differences between the two. The device 900 shown in Figure 10 further comprises a static port 992. The static port 992 is defined in a portion of the housing that defines the flow channel, and is located between the first and second pitot tubes 922, 932, and the mouthpiece. The static port 922 lies parallel to the first direction.

The device 900 therefore comprises a static port 992, denoted as the second port, the first pitot tube 922 comprising the first port 926, and the second pitot tube 932 comprising a third port 936.

The device 900 comprises a first sensor assembly, the first sensor assembly comprising a first pressure sensor 993 configured to measure the pressure in the first pitot tube 922, and a static pressure sensor 991 configured to measure the pressure at the static (second) port 992.

The device 900 comprises a second sensor assembly, the second sensor assembly comprising a second pressure sensor 994 configured to measure the pressure in the first pitot tube 922, and the static pressure sensor 991 configured to measure the pressure at the static (second) port 992. The first pressure sensor 993 is sensitive to a higher range of pressures than the second pressure sensor 994, so the first sensor assembly is sensitive to a higher range of pressures than the second sensor assembly.

The device 900 comprises a third sensor assembly, the third sensor assembly comprising a third pressure sensor 995 configured to measure the pressure in the second pitot tube 932, and the static pressure sensor 991 configured to measure the pressure at the static (second) port 992.

The device 900 comprises a fourth sensor assembly, the fourth sensor assembly comprising a fourth pressure sensor 996 configured to measure the pressure in the second pitot tube 932, and the static pressure sensor 991 configured to measure the pressure at the static (second) port 992. The third pressure sensor 995 is sensitive to a higher range of pressures than the fourth pressure sensor 996, so the third sensor assembly is sensitive to a higher range of pressures than the fourth sensor assembly.

When air flows in the air flow channel in the first direction, for example when a user exhales on the mouthpiece, the first and second sensor assembly is configured to measure the difference between the pressure in the first pitot tube 922 and the pressure at the static (second) port 992.

When air flows in the air flow channel in the second direction, for example when a user inhales on the mouthpiece, the third and fourth sensor assembly is configured to measure the difference between the pressure in the second pitot tube 932 and the pressure at the static (second) port 992.

Whilst in this embodiment, for example, the first sensor assembly comprises a first sensor configured to measure the pressure in the first pitot tube and a second sensor configured to measure the pressure at the second port, the first sensor assembly may instead comprise a first differential sensor configured to measure the differential pressure between the first pitot tube and the second port. Similarly the second sensor assembly may instead comprise a second differential sensor configured to measure the differential pressure between the first pitot tube and the second port. Similarly, the third or fourth sensor assemblies may instead comprise a third or fourth differential sensors configured to measure the differential pressures between the second pitot tube and the second port.

## Claims

1. A device (100; 900) for performing one or more fluid flow tests, the device (100; 900) comprising:
a housing (104, 106) defining a flow channel (110) having a distal end and a proximal end such that fluid flowing from the proximal end to the distal end flows in a first direction;
a first pitot tube (122; 922) comprising a first port (126; 926) in fluidic communication with the flow channel (110), the first port (126; 926) facing in a second direction that is opposite to the first direction;
a second port (136; 992) in fluidic communication with the flow channel (110), the second port not facing in the second direction;
a first sensor assembly for measuring the difference between the pressure at the first port (126; 926) and the pressure at the second port (136; 992); and
a second sensor assembly;
**characterised in that** the second sensor assembly is for measuring the difference between the pressure at the first port (126; 926) and the pressure at the second port (136; 992); and
wherein the first sensor assembly is sensitive to a range of pressure or differential pressure that is higher than the range that the second sensor assembly is sensitive to.

2. A device (100; 900) according to claim 1, wherein the device (100; 900) is for performing a first type of respiratory test and a second type of respiratory test, the first type of respiratory test being different to the second type of respiratory test.

3. A device (100; 900) according to claim 1 or 2, wherein the first sensor assembly comprises a differential pressure sensor (152) in fluidic communication with both the first port (126; 926) and the second port (136; 992) and the second sensor assembly comprises a differential pressure sensor (154) in fluidic communication with both the first port (126; 926) and the second port (136; 992).

4. A device (100; 900) according to claim 3, wherein the differential pressure sensor (152) of the first sensor assembly is sensitive to a maximum differential pressure that is higher than the maximum differential pressure that the differential pressure sensor (154) of the second sensor assembly is sensitive to.

5. A device (100) according to any one of the preceding claims, further comprising a second pitot tube (132) comprising the second port (136), wherein the first and second sensor assemblies are configured to measure the difference between the pressure in the first pitot tube (122) and the pressure in the second pitot tube (132).

6. A device (100) according to claim 5, wherein the second port (136) faces away from the second direction.

7. A device (100) according to any one of the preceding claims, wherein a longitudinal axis is defined through the centre of the flow channel (110) and wherein the first port (126) and the second port (136) are aligned with the longitudinal axis.

8. A device (100; 900) according to any one of the preceding claims, further comprising a flow lineariser (160) positioned within the flow channel (110) configured to reduce turbulent fluid flow immediately upstream and/or immediately downstream of the first pitot tube (122; 922) when fluid flows in the first direction.

9. A device (100) according to claim 8, wherein the flow lineariser (160) comprises a wall defining a conduit, a central axis of the conduit extending in a direction substantially parallel to the first direction, and wherein the first port (126) and the second port (136) are both received within the conduit.

10. A device (100; 900) according any one of the preceding claims, wherein the first pitot tube (122; 922) is removably couplable to the device housing (104, 106).

11. A device (100) according to any one of the preceding claims, further comprising a second pitot tube (132) comprising the second port (136) and wherein the second pitot tube is removably couplable to the device housing (104, 106), further comprising a pitot tube module (120) comprising the first pitot tube (122) and the second pitot tube (132) and wherein the pitot tube module (120) may be removably couplable from the device housing (104, 106) as a single unit, and further comprising a seal (184) between the pitot tube module and the device housing (104, 106).

12. A device (100) according to any one of the preceding claims, further comprising a sensor module (150) comprising the first and second sensor assemblies, wherein the sensor module is removably couplable to the device housing (104, 106) as a single unit, and further comprising a pitot tube module (120) comprising the first pitot tube (122) and a second pitot tube (132) comprising the second port (136), wherein the sensor module (150) is removably couplable to the pitot tube module (120).

13. A device (100; 900) according to any one of the preceding claims, further comprising control circuitry (171) configured to receive signals from the first and second sensor assemblies, wherein the device (100; 900) is configured or configurable for performing a first fluid flow test and wherein the control circuitry (171) is configured to measure or determine a pressure difference value during the first fluid flow test and to output a result for the first flow test based on the measured or determined dynamic pressure value, and wherein the device (100; 900) is configured or configurable for performing a second fluid flow test and wherein the control circuitry (171) is configured to output a result for the second flow test based on the measured or determined dynamic pressure value.

14. A method of performing a fluid flow test using a device (100; 900) according to any one of the preceding claims, the method comprises the step of: measuring or determining a difference between the pressure in the first pitot tube (122; 922) and the pressure at the second port (136; 992) using at least one of the first and second sensor assemblies.

15. A method according to claim 14, further comprising the step of performing a first fluid flow test using the device (100; 900) and outputting a result for the first flow test based on the measured or determined dynamic pressure value, and further the step of performing a second fluid flow test using the device (100; 900) and outputting a result for the second flow test based on the measured or determined dynamic pressure value.

## Patentansprüche

1. Vorrichtung (100; 900) zum Durchführen von einer oder mehreren Fluiddurchflussprüfungen, wobei die Vorrichtung (100; 900) aufweist:
ein Gehäuse (104, 106), das einen Strömungskanal (110) mit einem distalen Ende und einem proximalen Ende definiert, so dass Fluid, das vom proximalen Ende zum distalen Ende fließt, in einer ersten Richtung fließt;
ein erstes Pitotrohr (122; 922), das eine erste Öffnung (126; 926) aufweist, die in Fluidverbindung mit dem Strömungskanal (110) steht, wobei die erste Öffnung (126; 926) in eine zweite Richtung weist, die der ersten Richtung entgegengesetzt ist;
eine zweite Öffnung (136; 992), die in Fluidverbindung mit dem Strömungskanal (110) steht, wobei die zweite Öffnung nicht in die zweite Richtung weist;
eine erste Sensoranordnung zum Messen der Differenz zwischen dem Druck an der ersten Öffnung (126; 926) und dem Druck an der zweiten Öffnung (136; 992); und
eine zweite Sensoranordnung;
**dadurch gekennzeichnet, dass** die zweite Sensoranordnung zum Messen der Differenz zwischen dem Druck an der ersten Öffnung (126; 926) und dem Druck an der zweiten Öffnung (136; 992) vorgesehen ist; und
wobei die erste Sensoranordnung gegenüber einem Druck- oder Differenzdruckbereich empfindlich ist, der höher ist als der Bereich, demgegenüber die zweite Sensoranordnung empfindlich ist.

2. Vorrichtung (100; 900) nach Anspruch 1, wobei die Vorrichtung (100; 900) zum Durchführen einer ersten Art von Atemtest und einer zweiten Art von Atemtest konfiguriert ist, wobei sich die erste Art von Atemtest von der zweiten Art von Atemtest unterscheidet.

3. Vorrichtung (100; 900) nach Anspruch 1 oder 2, wobei die erste Sensoranordnung einen Differenzdrucksensor (152) aufweist, der sowohl mit der ersten Öffnung (126; 926) als auch der zweiten Öffnung (136; 992) in fluidischer Verbindung steht, und die zweite Sensoranordnung einen Differenzdrucksensor (154) aufweist, der sowohl mit der ersten Öffnung (126; 926) als auch der zweiten Öffnung (136; 992) in fluidischer Verbindung steht.

4. Vorrichtung (100; 900) nach Anspruch 3, wobei der Differenzdrucksensor (152) der ersten Sensoranordnung gegenüber einem maximalen Differenzdruck empfindlich ist, der höher ist als der maximale Differenzdruck, demgegenüber der Differenzdrucksensor (154) der zweiten Sensoranordnung empfindlich ist.

5. Vorrichtung (100) nach einem der vorhergehenden Ansprüche, die ferner ein zweites Pitotrohr (132) aufweist, das die zweite Öffnung (136) aufweist, wobei die erste und die zweite Sensoranordnung zum Messen der Differenz zwischen dem Druck im ersten Pitotrohr (122) und dem Druck im zweiten Pitotrohr (132) konfiguriert sind.

6. Vorrichtung (100) nach Anspruch 5, wobei die zweite Öffnung (136) der zweiten Richtung abgewandt ist.

7. Vorrichtung (100) nach einem der vorhergehenden Ansprüche, wobei eine Längsachse durch die Mitte des Strömungskanals (110) hindurch definiert ist und wobei die erste Öffnung (126) und die zweite Öffnung (136) auf die Längsachse ausgerichtet sind.

8. Vorrichtung (100; 900) nach einem der vorhergehenden Ansprüche, die ferner einen in dem Strömungskanal (110) angeordneten Strömungsgleichrichter (160) aufweist, der zum Verringern einer turbulenten Fluidströmung unmittelbar stromaufwärts und/oder unmittelbar stromabwärts des ersten Pitotrohrs (122; 922), wenn Fluid in der ersten Richtung fließt, konfiguriert ist.

9. Vorrichtung (100) nach Anspruch 8, wobei der Strömungsgleichrichter (160) eine Wand aufweist, die einen Leitkanal definiert, wobei eine Mittelachse des Leitkanals sich in einer zu der ersten Richtung im Wesentlichen parallelen Richtung erstreckt, und wobei die erste Öffnung (126) und die zweite Öffnung (136) beide im Leitkanal aufgenommen sind.

10. Vorrichtung (100; 900) nach einem der vorhergehenden Ansprüche, wobei das erste Pitotrohr (122; 922) abnehmbar mit dem Vorrichtungsgehäuse (104, 106) koppelbar ist.

11. Vorrichtung (100) nach einem der vorhergehenden Ansprüche, die ferner ein zweites Pitotrohr (132) aufweist, das die zweite Öffnung (136) aufweist, und wobei das zweite Pitotrohr abnehmbar mit dem Vorrichtungsgehäuse (104, 106) koppelbar ist, die ferner ein Pitotrohrmodul (120) aufweist, das das erste Pitotrohr (122) und das zweite Pitotrohr (132) aufweist, und wobei das Pitotrohrmodul (120) als einzelne Einheit vom Vorrichtungsgehäuse (104, 106) abnehmbar koppelbar ist, und die ferner eine Dichtung (184) zwischen dem Pitotrohrmodul und dem Vorrichtungsgehäuse (104, 106) aufweist.

12. Vorrichtung (100) nach einem der vorhergehenden Ansprüche, die ferner ein Sensormodul (150) aufweist, das die erste und die zweite Sensoranordnung aufweist, wobei das Sensormodul als einzelne Einheit abnehmbar mit dem Vorrichtungsgehäuse (104, 106) koppelbar ist, und die ferner ein Pitotrohrmodul (120) aufweist, das das erste Pitotrohr (122) und ein zweites Pitotrohr (132), das die zweite Öffnung (136) aufweist, aufweist, wobei das Sensormodul (150) abnehmbar mit dem Pitotrohrmodul (120) koppelbar ist.

13. Vorrichtung (100; 900) nach einem der vorhergehenden Ansprüche, die ferner eine Steuerschaltungsanordnung (171) aufweist, die zum Empfangen von Signalen von der ersten und der zweiten Sensoranordnung konfiguriert ist, wobei die Vorrichtung (100; 900) zum Durchführen einer ersten Fluiddurchflussprüfung konfiguriert oder konfigurierbar ist und wobei die Steuerschaltungsanordnung (171) zum Messen oder Ermitteln eines Druckdifferenzwerts während der ersten Fluiddurchflussprüfung und zum Ausgeben eines Ergebnisses für die erste Durchflussprüfung auf Basis des gemessenen oder ermittelten Staudruckwerts konfiguriert ist und wobei die Vorrichtung (100; 900) zum Durchführen einer zweiten Fluiddurchflussprüfung konfiguriert oder konfigurierbar ist und wobei die Steuerschaltungsanordnung (171) zum Ausgeben eines Ergebnisses für die zweite Durchflussprüfung auf Basis des gemessenen oder ermittelten Staudruckwerts konfiguriert ist.

14. Verfahren zur Durchführen einer Fluiddurchflussprüfung unter Verwendung einer Vorrichtung (100; 900) nach einem der vorhergehenden Ansprüche, wobei das Verfahren den folgenden Schritt aufweist: Messen oder Ermitteln einer Differenz zwischen dem Druck in dem ersten Pitotrohr (122; 922) und dem Druck an der zweiten Öffnung (136; 992) unter Verwendung von mindestens einer von der ersten und der zweiten Sensoranordnung.

15. Verfahren nach Anspruch 14, das ferner den Schritt des Durchführens einer ersten Fluiddurchflussprüfung unter Verwendung der Vorrichtung (100; 900) und des Ausgebens eines Ergebnisses für die erste Durchflussprüfung auf Basis des gemessenen oder ermittelten Staudruckwerts und ferner den Schritt des Durchführens einer zweiten Fluiddurchflussprüfung unter Verwendung der Vorrichtung (100; 900) und des Ausgebens eines Ergebnisses für die zweite Fluiddurchflussprüfung auf Basis des gemessenen oder ermittelten Staudruckwerts aufweist.

## Revendications

1. Dispositif (100 ; 900) destiné à réaliser un ou plusieurs tests d'écoulement de fluide, le dispositif (100 ; 900) comprenant :
un boîtier (104, 106) définissant un canal d'écoulement (110) présentant une extrémité distale et une extrémité proximale telles qu'un fluide s'écoulant de l'extrémité proximale à l'extrémité distale s'écoule dans une première direction ;
un premier tube de Pitot (122 ; 922) comprenant un premier orifice (126 ; 926) en communication fluidique avec le canal d'écoulement (110), le premier orifice (126 ; 926) étant orienté dans une seconde direction opposée à la première direction ;
un second orifice (136 ; 992) en communication fluidique avec le canal d'écoulement (110), le second orifice n'étant pas orienté dans la seconde direction ;
un premier ensemble de capteurs destiné à mesurer la différence entre la pression au niveau du premier orifice (126 ; 926) et la pression au niveau du second orifice (136 ; 992) ; et
un second ensemble de capteurs ;
**caractérisé en ce que** le second ensemble de capteurs sert à mesurer la différence entre la pression au niveau du premier orifice (126 ; 926) et la pression au niveau du second orifice (136 ; 992) ; et
dans lequel le premier ensemble de capteurs est sensible à une plage de pression ou de pression différentielle supérieure à la plage à laquelle le second ensemble de capteurs est sensible.

2. Dispositif (100 ; 900) selon la revendication 1, le dispositif (100 ; 900) servant à réaliser un premier type de test respiratoire et un second type de test respiratoire, le premier type de test respiratoire étant différent du second type de test respiratoire.

3. Dispositif (100 ; 900) selon la revendication 1 ou 2, dans lequel le premier ensemble de capteurs comprend un capteur de pression différentielle (152) en communication fluidique avec à la fois le premier orifice (126 ; 926) et le second orifice (136 ; 992) et le second ensemble de capteurs comprend un capteur de pression différentielle (154) en communication fluidique avec à la fois le premier orifice (126 ; 926) et le second orifice (136 ; 992).

4. Dispositif (100 ; 900) selon la revendication 3, dans lequel le capteur de pression différentielle (152) du premier ensemble de capteurs est sensible à une pression différentielle maximale supérieure à celle à laquelle le capteur de pression différentielle (154) du second ensemble de capteurs est sensible.

5. Dispositif (100) selon l'une quelconque des revendications précédentes, comprenant en outre un second tube de Pitot (132) comprenant le second orifice (136), dans lequel les premier et second ensembles de capteurs sont configurés pour mesurer la différence entre la pression dans le premier tube de Pitot (122) et la pression dans le second tube de Pitot (132).

6. Dispositif (100) selon la revendication 5, dans lequel le second orifice (136) est orienté à l'opposé de la seconde direction.

7. Dispositif (100) selon l'une quelconque des revendications précédentes, dans lequel un axe longitudinal est défini par le centre du canal d'écoulement (110) et dans lequel le premier orifice (126) et le second orifice (136) sont alignés avec l'axe longitudinal.

8. Dispositif (100) selon l'une quelconque des revendications précédentes, comprenant en outre un linéariseur d'écoulement (160) positionné dans le canal d'écoulement (110) configuré pour réduire un écoulement de fluide turbulent immédiatement en amont et/ou immédiatement en aval du premier tube de Pitot (122 ; 922) lorsque le fluide s'écoule dans la première direction.

9. Dispositif (100) selon la revendication 8, dans lequel le linéariseur d'écoulement (160) comprend une paroi définissant un conduit, un axe central du conduit s'étendant dans une direction sensiblement parallèle à la première direction, et dans lequel le premier orifice (126) et le second orifice (136) sont tous deux reçus à l'intérieur du conduit.

10. Dispositif (100 ; 900) selon l'une quelconque des revendications précédentes, dans lequel le premier tube de Pitot (122 ; 922) peut être couplé de manière amovible au boîtier (104, 106) du dispositif.

11. Dispositif (100) selon l'une quelconque des revendications précédentes, comprenant en outre un second tube de Pitot (132) comprenant le second orifice (136) et dans lequel le second tube de Pitot peut être couplé de manière amovible au boîtier (104, 106) du dispositif, comprenant en outre un module de tubes de Pitot (120) comprenant le premier tube de Pitot (122) et le second tube de Pitot (132) et dans lequel le module de tubes de Pitot (120) peut être couplé de manière amovible à partir du boîtier (104, 106) du dispositif en tant qu'unité unique, et comprenant en outre un joint (184) entre le module de tubes de Pitot et le boîtier (104, 106) du dispositif.

12. Dispositif (100) selon l'une quelconque des revendications précédentes, comprenant en outre un module de capteurs (150) comprenant les premier et second ensembles de capteurs, dans lequel le module de capteurs peut être couplé de manière amovible au boîtier (104, 106) du dispositif en tant qu'unité unique, et comprenant en outre un module de tubes de Pitot (120) comprenant le premier tube de Pitot (122) et un second tube de Pitot (132) comprenant le second orifice (136), dans lequel le module de capteurs (150) peut être couplé de manière amovible au module de tubes de Pitot (120).

13. Dispositif (100) selon l'une quelconque des revendications précédentes, comprenant en outre des circuits de commande (171) configurés pour recevoir des signaux à partir des premier et second ensembles de capteurs, dans lequel le dispositif (100 ; 900) est configuré ou configurable pour réaliser un premier test d'écoulement de fluide et dans lequel les circuits de commande (171) sont configurés pour mesurer ou déterminer une valeur de différence de pression durant le premier test d'écoulement de fluide et délivrer un résultat du premier test d'écoulement basé sur la valeur de pression dynamique mesurée ou déterminée, et le dispositif (100 ; 900) étant configuré ou configurable pour réaliser un second test d'écoulement de fluide et dans lequel les circuits de commande (171) sont configurés pour délivrer un résultat du second test d'écoulement de débit basé sur la valeur de pression dynamique mesurée ou déterminée.

14. Procédé de réalisation d'un test d'écoulement de fluide à l'aide d'un dispositif (100 ; 900) selon l'une quelconque des revendications précédentes, le procédé comprenant l'étape de : mesure ou détermination d'une différence entre la pression dans le premier tube de Pitot (122 ; 922) et la pression au niveau du second orifice (136 ; 992) à l'aide d'au moins un des premier et second ensembles de capteurs.

15. Procédé selon la revendication 14, comprenant en outre l'étape de réalisation d'un premier test d'écoulement de fluide à l'aide du dispositif (100 ; 900) et délivrer un résultat du premier test d'écoulement basé sur la valeur de pression dynamique mesurée ou déterminée, et en outre l'étape de réalisation d'un second test d'écoulement de fluide avec le dispositif (100 ; 900) et la délivrance d'un résultat du second test d'écoulement basé sur la valeur de pression dynamique mesurée ou déterminée.
